# EUROPEAN PATENT APPLICATION

(11) **EP 4 424 701 A1**
(43) Date of publication of application: **04.09.2024**
(21) Application number: 22897679.1
(22) Date of filing: 16.11.2022
(51) Int. Cl.: C07K 7/64, C07K 11/02, C07K 14/00, A61K 38/12, A61K 38/16, A61P 1/00, A61P 5/00, A61P 9/00, A61P 11/00, A61P 19/00, A61P 25/00, A61P 27/02, A61P 27/16, A61P 31/04, A61P 31/12, A61P 37/04

(54) **CYCLIC POLYPEPTIDE CARRIER FOR EFFICIENT DELIVERY OF NUCLEIC ACID, AND VARIANTS THEREOF**

(30) Priority: 25.11.2021 CN 202111412266
(71) Applicant: Suzhou Healirna Biotechnology Co., Ltd., Suzhou, Jiangsu 215000 (CN)
(72) Inventor: ZHAO, Lixiang, Suzhou, Jiangsu 215000 (CN); LU, Qingqing, Suzhou, Jiangsu 215000 (CN); FAN, Yawen, Suzhou, Jiangsu 215000 (CN); YI, Cheng, Suzhou, Jiangsu 215000 (CN); GE, Youzhen, Suzhou, Jiangsu 215000 (CN); XIA, Dan, Suzhou, Jiangsu 215000 (CN); HU, Hongpeng, Suzhou, Jiangsu 215000 (CN)
(74) Representative: Sun, Yiming
(86) International application number: PCT/CN2022/132290
(87) International publication number: WO 2023/093596

(57) **Abstract**

The present invention relates to a novel cyclic peptide carrier in the field of pharmaceutical technology and its various forms, where the cyclic peptide has the general sequence formula P-(X)m-Cys-(Y)n-Cys-(Z)o-P. The invention also encompasses the construction and preparation technology of a composition or formulation formed by the novel cyclic peptide carrier carrying nucleic acids, drugs, or therapeutic agents. Furthermore, it involves a method for effectively delivering nucleic acids to cells, tissues, or organisms using the composition or formulation, as well as its medical applications.

## Description

### Field of the Invention

The present invention pertains to the technical field of pharmaceutical technology, involving a cyclic peptide carrier and its variants, as well as the construction and preparation techniques for compositions or formulations where the cyclic peptide carrier transports nucleic acids, drugs, or therapeutic agents. Additionally, the invention relates to methods for effectively delivering nucleic acids into cells, tissues, or organisms using the compositions or formulations comprised of the cyclic peptide carrier and its variants, along with their medical applications.

### Background technology

Molecular medical therapies that involve the introduction of specific or normal nucleic acid sequences into patients' cells or tissues are increasingly being popularized in the prevention, treatment, and improvement of human diseases. Nucleic acid drugs theoretically possess effects that traditional drugs cannot replace; they not only enter cells like small molecule drugs to exert their function but also possess precise target specificity like antibody drugs. Modern medical development has solved a variety of human disease issues, but there are still a significant number of genetic diseases that are almost untreatable in current clinical therapy, such as hereditary diseases, metabolic diseases, tumors, and viral infectious diseases (Front Oncol. 2019 Apr 24; 9:297. and Am J Med Genet A. 2017 Sep; 173(9):2307-2322).

Nucleic acid drugs have special advantages in these diseases; they can not only engage in personalized disease development by targeting genetic differences or expression abnormalities but also through large-scale disease development via the introduction of exogenous genes encoding certain antigen proteins (Cell. 2021 Mar 18; 184(6): 1589-1603). The current main categories of nucleic acid drugs include chemically synthesized oligonucleotides, such as small interfering nucleic acids siRNA, micro nucleic acids microRNA, antisense nucleic acids, aptamers, and ribonucleic acid drugs produced by biotechnology, such as messenger mRNA therapeutic drugs and mRNA vaccines.

Despite the numerous advantages of nucleic acid drug technology and gene therapy, if nucleic acid molecules are not chemically modified or protected by carriers, their highly negative charge and hydrophilic physicochemical properties make them very susceptible to degradation by the body or cells, thus failing to achieve the therapeutic effect of the drug (J Control Release. 2015 Nov 10; 217:345-51). Therefore, the main technical barrier to the development of nucleic acid drugs lies in the delivery system, that is, how to remain intact for sufficient time after drug injection to accurately target the lesion site and avoid harming normal cells (J Control Release. 2015 Nov 10; 217:345-51). From a technical development perspective, one approach is to chemically modify the nucleic acids, ensuring the stability and activity of the nucleic acids during transportation (Nat Rev Drug Discov. 2014 Oct; 13(10):759-80). Modified nucleic acid molecules need to consider various properties, primarily including stability, pharmacokinetic properties, base pairing affinity, avoiding the immune system while ensuring recognition by functional enzymes since drugs like ASO, siRNA, and mRNA all require the action of various enzymes (RNase, Ago2, and RNAPolymerase) to be effective (Postdoc J. 2016 Jul; 4(7):35-50). The quantities and combinations of various modifications greatly impact the drug-like properties of a molecule; a specific modification might cause the loss of enzyme recognition. Therefore, in drug design, it is necessary to repeatedly design and test to identify the most rational combination. Another approach is to design and develop drug carriers that bind to nucleic acids, ensuring that the nucleic acids can reach target cells/organs stably and efficiently (Molecular Therapy, 2019, 27(4):710-728).

Over the past few decades, various synthetic or naturally existing compounds such as liposomes, polymers, proteins, and peptides have been developed as carrier systems for nucleic acid drugs (Mol Ther. 2019 Apr 10; 27(4):710-728). Their main feature is to form complexes by the electrostatic attraction between the positively charged parts and the negatively charged parts of the nucleic acids, compressing and encapsulating the nucleic acids internally to prevent degradation. For example, by combining nucleic acids with cationic liposomes, and then introducing auxiliary lipids to form stable liposomal bilayer structure particles - Lipid NanoParticles (LNPs) that encapsulate nucleic acids inside. The FDA-approved LNP delivery system consists of ionizable cationic lipids, phospholipids, cholesterol, and polyethylene glycol (PEG) lipids, whose formulation components and molar ratios have been optimized multiple times to ensure a certain efficiency of liver delivery (Curevac, BioNTech, Moderna).

Although LNPs have greatly promoted the development of nucleic acid drugs, there is still room for improvement. The nanoparticles are large and unstable, generally requiring low temperature or even sub-zero temperature storage. They can usually only be effectively introduced into tissues with large spaces, such as the liver, spleen, and tumors, through intravenous injection (Int J Pharm. 2021 May 15; 601:120586). Additionally, the allergic reactions of LNPs are relatively severe and require the use of antihistamines and steroid medications before injection. These drawbacks limit LNP drugs to serious diseases such as rare diseases and cancer.

Therefore, efficiently delivering nucleic acids into the tissues and organs of the body for targeted therapy related to gene regulation remains a critical issue to be resolved. Apart from the LNP delivery system, many other types of delivery systems have been developed, such as polymers or their derivatives, peptides, and proteins (Life (Basel). 2022 Aug 17; 12(8):1254). Linear or branched polyethylenimine (PEI) and amino polysters (APE) are widely used cationic polymers for in vivo and in vitro nucleic acid delivery (J Control Release. 2012 Jul 20; 161 (2):554-65). Compared to lipids, polymers are less used in nucleic acid therapy, largely due to the complexity of their molecules and the uncontrollability of manufacturing (J. Am. Chem. Soc. 2021, 143, 20529-20545). Simple and effective polymers are most likely to be used clinically, such as polyamidoamine (PAMAM) or polypropyleneimine-based dendrimers. At the same time, biosafety and bioresponsiveness are key factors to consider in nucleic acid delivery. The former aids in reducing toxicity and enhances the therapeutic index, while the latter may promote cellular uptake and bioresponsive endosomal escape (J Control Release. 2015 Nov 10; 217:345-51. and J. Am. Chem. Soc. 2021, 143, 20529 20545).

Proteins and peptides are also commonly used materials for nucleic acid delivery. Cell-penetrating peptides can also deliver mRNA (Pharmaceutics2022,14(1),78). Cell-penetrating peptides include cationic peptides, amphipathic peptides, and hydrophobic peptides that can be flexibly designed and synthesized by solid-phase peptide synthesis. Most cell-penetrating peptides contain cationic amino acid residues, such as lysine (Lys) or arginine (Arg). Peptides have unique features in that they can be simply and flexibly designed, possess various functionalities, and allow precise control of the composition of each functional unit of the peptide. Additionally, peptides have monodisperse characteristics, which is a good component for synthesizing nanoparticles as they allow control over the size of nanoparticles and the quantity of cargo. Cell-penetrating peptides can exhibit a considerable ability for cell penetration and reduced cytotoxicity. Representative examples include protamine, a small protein rich in arginine that can tightly bind to mRNA to form nanoparticles, and has entered clinical trials. However, protamine has the issue of being too tightly wrapped and difficult to release. Cell-penetrating peptides (CPPs) are a class of small molecular peptides with strong transmembrane transport capabilities that can carry various macromolecules such as peptides, proteins, and nucleic acids into cells. The amino acid residue count of CPPs is usually no more than 30 and is rich in basic amino acids. The cellular transport mechanism of CPPs is generally considered to include three types: direct cell entry via electrostatic interactions, transduction domains, and endocytosis. There are many types and research on cell-penetrating peptides, such as naturally occurring HIV viral proteins and apamin-derived Tat peptides and apamin, as well as artificially designed and synthesized polymers of arginine, lysine, Pap-1, MPG peptides, etc. While cell-penetrating peptides currently have broad application prospects as emerging drug carriers, CPPs also have their shortcomings, such as poor stability, the efficiency of targeting tissues or organs, and accumulation problems that need further resolution. In the field of peptide drug development, to overcome the difficulties of short half-life, the structural robustness, cell transmembrane property, and metabolic stability of peptide molecules can be effectively improved through different amino acid regular arrangements and chemical coupling, which can be referred to the structural design strategy of cell-penetrating peptides.

Peptides contain one or more nucleic acid binding sites composed of cationic amino acids (arginine, lysine, or histidine) and also include a hydrophobic part composed of hydrophobic amino acids, which can stabilize the peptide nucleic acid complex and enhance cell penetration. The distribution of cationic and hydrophobic amino acids determines the secondary structure of peptides in aqueous solutions and the conformational change of peptides after interacting with nucleic acids. These factors collectively determine the complexation strength between peptide and nucleic acid, self-assembly, and membrane-penetrating ability, as well as the capacity to protect and release nucleic acids, thereby determining the gene delivery effect. Cyclic cell-penetrating peptides have a low entropy value of cyclic conformation, are enzyme-resistant, and after cyclization, the rotation of the peptide chain linkage is restricted, reducing freedom and increasing stability. The conformation is more easily analyzed, and the likelihood of binding with the receptor is significantly increased. Cyclic cell-penetrating peptides are mainly synthesized by three methods: backbone to backbone cyclization; backbone to side chain cyclization; and side chain to side chain cyclization. The backbone to backbone is connected by the amide bond between N terminus and C terminus, the backbone to side chain cyclization forms an amide bond between the backbone N end and the side chain carboxylic acids of Asp or Glu, and the side chain to side chain cyclization can be formed by amide or disulfide bonds.

Cyclic peptides serve as both drugs and research tools with many good properties (Chem. Rev. 2019, 119, 17, 10241-10287). First, the cyclization of peptides reduces conformational freedom, greatly improving metabolic stability and binding affinity for target molecules; second, cyclic peptides possess immense structural diversity. Using just these 20 proteinogenic amino acids, different cyclic peptides can be generated; lastly, compared to proteins, cyclic peptides retain some characteristics of small molecules, such as stability, lower risk of immune response, synthesizability, and lower production costs.

Peptide-based carriers have become promising candidates for gene delivery due to their versatility, ease of synthesis, good biocompatibility, and low cytotoxicity. The biocompatibility and multifunctionality of peptides make them an ideal carrier for gene delivery. Furthermore, by incorporating functional sequences of bioactive peptides into synthetic peptides, bioactive peptide carriers can be generated. Examples of such biofunctionalization include the combining cell-penetrating peptide sequences to enhance cellular absorption and combining bio-recognition elements for specific tissue targeting. Intracellular targeting by peptide-DNA or peptide-RNA nano-complexes interacting with intracellular targets is another possible method to improve the selectivity and efficiency of gene therapy. Most peptide-based gene carriers developed to date are still unable to match the transfection efficiency of commercially available transfection reagents, and relatively few peptide carriers are currently considered for therapeutic use. This indicates that through rational design and structure-activity relationship studies, further development of peptide-based carriers with higher transfection efficiency is needed.

In summary, how to safely and efficiently introduce specific or normal nucleic acid sequences into the cells or tissues of patients or animals through molecular medical therapy, in the prevention, treatment, and improvement of human or animal diseases, is an important technical issue worthy of continued development to provide a variety of optimized solutions.

### SUMMARY OF THE INVENTION

In the context of the background described, the present invention introduces a novel type of cyclic peptide carrier and its variable forms that possess high gene delivery efficiency, stable structure, and optimal biocompatibility. The technical solutions are as follows:
In one instance, the invention presents a cyclic peptide with the sequence general formula: P-(X)m-Cys-(Y)n-Cys-(Z)o-P; wherein,
X = Xaa₁-Xaa₂-Leu, or Xaa₁-Xaa₂-Xaa₃-Leu, or Xaa₁-Xaa₂-Xaa₃-Xaa₄-Leu;
Y = Xaa₅-Xaa₆-Lys-Xaa₆-Xaa₅, or Xaa₅-Xaa₆-Xaa₇-Lys-Xaa₇-Xaa₆-Xaa₅, or Xaa₅-Xaa₆-Xaa₇-Xaa₈-Lys-Xaa₈-Xaa₇-Xaa₆-Xaa₅-,
Z= Leu-Xaa₂-Xaa₁, or Leu-Xaa₃-Xaa₂-Xaa₁, or Leu-Xaa₄-Xaa₃-Xaa₂-Xaa₁.
Xaa₁, Xaa₂, Xaa₃, Xaa₄, Xaa₅, Xaa₆, Xaa₇, and Xaa₈ are each independently selected from the group of amino acids consisting of alanine (Ala), valine (Val), leucine (Leu), isoleucine (Ile), proline (Pro), phenylalanine (Phe), tryptophan (Trp), methionine (Met), glycine (Gly), serine (Ser), tyrosine (Tyr), threonine (Thr), asparagine (Asn), glutamine (Gln), aspartic acid (Asp), glutamic acid (Glu), lysine (Lys), or arginine (Arg).

P is an optional ligand, which can be present or absent, and can be independently selected from entities such as spermine or spermidine, polylysine, polyarginine, MPG peptide, Tat peptide associated with HIV, SAP peptide, MAP peptide, KALA peptide, FGF peptide, HSV peptide, RGD peptide, GLP-1 peptide, Pep-1 peptide, signal peptides or sequences, cytokines, growth factors, hormones, small molecule drugs, carbohydrates, therapeutically active proteins or peptides, antigens or epitopes, antibodies, ligands for antibody receptors, synthetic ligands, receptor inhibitors or antagonists, immunostimulatory proteins or peptides, fish sperm protein, nucleolin, transferrin, polycationic peptide sequences, polyamines, peptide nucleic acids, carboxylated spermine, carboxylated spermidine, spermine and spermidine analogues and nucleic acid embedders, nuclear localization signals or sequences (NLS), cell-penetrating peptides, TAT, saccharides, mannose, galactose, small molecule agonists, poly-L-lysine (PLL), basic peptides, calcitonin peptides, FGF, lactoferrin, histones, peptides derived from or analogous to VP22, VP22 (Herpes Simplex).

A disulfide bond is formed between the two cysteines (Cys), and m, n, or o can each be independently chosen from the numbers 0, 1, 2, 3, 4, 5, 6, 7, 8, 9, or 10.

The novel cyclic peptide comprises a chain length ranging from 15 to 150 amino acid residues.

According to the present invention, the cyclic peptide of the general formula provided can be used on its own or in various combinations.

Preferably, the cyclic peptide or mixtures thereof can further be used to load nucleic acid molecules, therapeutic drugs or agents to form compositions, wherein the drug or agent is selected from peptides, antibodies, enzymes, hormones, antitumor drugs, antilipemic drugs, antibiotics, anti-inflammatory agents, cytotoxins, cell growth inhibitors, immunoregulators or neuroactive agents.

Preferably, the nucleic acid is characterized by including one or more selected from the group consisting of DNA or RNA, encoding DNA, encoding RNA, encoding mRNA, small interfering nucleic acid siRNA, small activating nucleic acid saRNA, micro nucleic acid microRNA, messenger nucleic acid mRNA, antisense nucleic acid, or immunostimulatory nucleic acid.

On the other hand, the present invention also provides any of the aforementioned cyclic peptides or cyclic peptide compositions, which are used in combination with one or more surfactants, liposomes, liposome-like entities, alcosomes, transporters, or phospholipid mixtures.

The cyclic peptide or cyclic peptide composition can be combined with polyethylene glycolated lipids, cationic lipids, neutral lipids, cationic polymers, targeting moieties, etc.The cyclic peptide or cyclic peptide composition can optionally be combined with one or more auxiliary lipids (i.e., neutral lipids, cholesterol or cholesterol derivatives, hemolytic lipids, or cationic lipids), or with one or more cationic lipids, or with one or more anionic lipids, or with one or more pH-sensitive lipid vesicle compositions.

Preferably, the liposomes characteristic of the present invention include, but are not limited to, N-(2,3-di(tetradecanoyloxy)propyl)-N,N,N-trimethylammonium chloride (DOTAP), N-(2,3-dioleyloxy-propyl)-N,N,N-trimethylammonium chloride (DOTMA), distearoylphosphatidylcholine (DSPC), dioleoylphosphatidylcholine (DOPC), dipalmitoylphosphatidylcholine (DPPC), dioleoylphosphatidylglycerol (DOPG), dioleoylphosphatidylethanolamine (DOPE), 3β-[N-(N',N'-dimethylam inoethane)carbamoyl]cholesterol (DC-chol), dimyristoylphosphatidylcholine (DMPC), dimyristoylphosphatidylethanolamine (DMPE), dioleoyldimethylammonium chloride (DODAC), 1-palmitoyl-2-oleoyl-sn-glycero-3-phosphocholine (POPC), 1,2-dioleoyl-3-trimethylammonium-propane (DOTAP-CI), 1,2-dioleyl-3-dimethylaminopropane (DODMA), 1,2-dimyristoyl-rac-glycerol methoxy polyethylene glycol (DMG-PEG2000), 2-[(spermine carbamoylamino)ethyl]-N,N-dimethyl-3-amino propane trifluoroacetate (DOSPA), 1,2-dioleoyl-3-dimethylammonium-propane (DODAP), 2,3-dioleoyloxy-N-[2-(spermine carboxamido)ethyl]-N,N-dimethyl-1-propanaminium trifluoroacetate (DOSPA), 28-carbamoylaminospermine carbamoylglutamate (DOGS), dioleoyl ethylphosphatidylcholine (DOEPC), diphphytanoylphosphatidylethanolamine (DPhPE), dipalmitoylphosphatidylethanolamine (DPPE), 3-β-[1-(aminoiminomethyl)amino]cholesterol (O-Chol), palmitoyloleoylphosphatidylethanolamine (POPE), dipalmitoyloleoylphosphatidyl-glycerol (DPPG), among other commercial liposomes, or any mixture of the aforementioned liposomes.

Preferably, in certain embodiments, the cyclic peptides or cyclic peptide compositions provided by the present invention can form complex particles with one or more of the types of liposomes mentioned above.

In another aspect, the present invention also provides the aforementioned cyclic peptide or cyclic peptide compositions, which include use in combination with one or more pharmaceutically acceptable carriers, buffers, diluents, excipients.

The carrier is employed in a solid or liquid form, depending upon the method of administration. Routes of administration include, without limitation, intradermal or transdermal, oral, parenteral-encompassing subcutaneous, intramuscular, or intravenous injections, as well as topical or intranasal among others. Suitable doses of the compositions (drugs) according to the present invention can be determined through conventional experiments using animal models. Such models include humans and animals, comprising, but not limited to, pets such as members of the Felidae or Canidae families; farm animals such as but not limited to cows, horses, goats, sheep, and pigs; research animals like mice, rats, rabbits, goats, sheep, pigs, dogs, cats, etc.; avians such as chickens, turkeys, and songbirds among others; and groupings of humans, goats, cows, pigs, dogs, cats, monkeys, apes, or gnawing mammals-which include mice, hamsters, and rabbits.

The present invention's cyclic peptides or cyclic peptide compositions can be utilized in preparations for the prevention, treatment, or amelioration of diseases selected from the following list: cancer or tumorous diseases, viral or bacterial infectious diseases, genetic disorders, respiratory system diseases, cardiovascular diseases, neurological disorders, digestive system conditions, skeletal disorders, connective tissue diseases, immunodeficiency disorders, endocrine disorders, and diseases of the eyes and ears.

The aforementioned cyclic peptides or cyclic peptide compositions of the present invention can also be employed for veterinary purposes, typically as drug compositions or for vaccination applications.

The cyclic peptide or cyclic peptide compositions of the present invention are capable of delivering RNA into the cells, tissues, or individuals of animals/humans, packaged within a kit.

In another embodiment, the present invention provides a cyclic peptide with the following sequence general formula:

P-(X)m-Cys-(Y)n-Cys-(Z)o-P;

wherein, X = Xaa₁-Xaa₂-Leu, or Xaa₁-Xaa₂-Xaa₃-Leu, or Xaa₁-Xaa₂-Xaa₃-Xaa₄-Leu. Y consists of a peptide segment composed of a variety of amino acids, in which the number of Lys within Y is from 1-20.
Z = Leu-Xaa₅-Xaa₆ or Leu-Xaa₅-Xaa₆-Xaa₇, or Leu-Xaa₅-Xaa₆-Xaa₇-Xaa₈.
Y = Xaa₅-Xaa₆-Lys-Xaa₆-Xaa₅,orXaa₅-Xaa₆-Xaa₇-Lys-Xaa₇-Xaa₆-Xaa₅, or Xaa₅-Xaa₆-Xaa₇-Xaa₈-Lys-Xaa₈-Xaa₇-Xaa₆-Xaa₅.
Z = Leu-Xaa₂-Xaa₁, or Leu-Xaa₃-Xaa₂-Xaa₁, or Leu-Xaa₄-Xaa₃-Xaa₂-Xaa₁.
Xaa₁, Xaa₂, Xaa₃, Xaa₄, Xaa₅, Xaa₆, Xaa₇, and Xaa₈ are alanine (Ala), valine (Val), leucine (Leu), isoleucine (Ile), proline (Pro), phenylalanine (Phe), tryptophan (Trp), methionine (Met), glycine (Gly), serine (Ser), tyrosine (Tyr), threonine (Thr), asparagine (Asn), glutamine (Gln), aspartic acid (Asp), glutamic acid (Glu), lysine (Lys) or arginine (Arg);Xaax consists of one or more amino acids composed of alanine (Ala), valine (Val), leucine (Leu), isoleucine (Ile), proline (Pro), phenylalanine (Phe), tryptophan (Trp), methionine (Met), glycine (Gly), serine (Ser), tyrosine (Tyr), threonine (Thr), asparagine (Asn), glutamine (Gln), aspartic acid (Asp), glutamic acid (Glu), lysine (Lys), or arginine (Arg).
Xaax is a peptide fragment composed of one or more amino acids selected from alanine (Ala), valine (Val), leucine (Leu), isoleucine (Ile), proline (Pro), phenylalanine (Phe), tryptophan (Trp), methionine (Met), glycine (Gly), serine (Ser), tyrosine (Tyr), threonine (Thr), asparagine (Asn), glutamine (Gln), aspartic acid (Asp), glutamic acid (Glu), lysine (Lys), or arginine (Arg).

P is an optional ligand that can be present or absent and can be independently selected from entities such as spermine or spermidine, polylysine, polyarginine, MPG peptide, HIV-binding peptide Tat, SAP peptide, MAP peptide, KALA peptide, FGF peptide, HSV peptide, RGD peptide, GLP-1 peptide, Pep-1 peptide, signal peptides or sequences, cytokines, growth factors, hormones, small molecule drugs, carbohydrates, therapeutically active proteins or peptides, antigens or epitopes, antibodies, ligands for antibody receptors, synthetic ligands, inhibitors or antagonists of receptors, immunostimulatory proteins or peptides.

A disulfide bond is formed between the two cysteines (Cys), and m, n, or o can be independently zero or any integer from 1 to 10.

The cyclic peptides contain lengths ranging from 15 to 150 amino acid residues.

The present invention provides that the cyclic peptide of the formula described herein can be used either independently or in combination with others.

Moreover, the present invention provides a cyclic peptide complex comprising the aforementioned cyclic peptide, wherein the sequence formula of the cyclic peptide is as follows:
P-(X)m-Cys-(Y)n-Cys-(Z)o-P, with a disulfide bond formed between the two cysteines (Cys).

Additionally, the present invention provides a cyclic peptide and drug composition, where the cyclic peptide has the peptide structure of the aforementioned scheme, which is capable of loading nucleic acid molecules, therapeutic drugs or agents to form compositions, with the drug or agent being one or more selected from peptides, antibodies, enzymes, hormones, antitumor drugs, antilipemic drugs, antibiotics, anti-inflammatory agents, cytotoxins, cell growth inhibitors, immunoregulators, or neuroactive agents.

Preferably, the nucleic acid being loaded includes one or more selected from DNA or RNA, encoding DNA, encoding RNA, encoding mRNA, small interfering nucleic acid siRNA, small activating nucleic acid saRNA, micro nucleic acid microRNA, messenger nucleic acid mRNA, antisense nucleic acid, or immunostimulatory nucleic acid.

Preferably, the cyclic peptide and drug composition can be combined with one or more surfactants, liposomes, liposome-like entities, alcosomes, transporters, phospholipids mixtures.The cyclic peptide or cyclic peptide composition can be combined with polyethylene glycolated lipids, cationic lipids, neutral lipids, cationic polymers, targeting moieties, etc.

The cyclic peptide or cyclic peptide composition can be optionally combined with one or more auxiliary lipids (i.e., neutral lipids, cholesterol or cholesterol derivatives, hemolytic lipids, or cationic lipids), or with one or more cationic lipids, or with one or more anionic lipids, or with lipid vesicle compositions of one or more pH-sensitive lipids.

Preferably, the liposomes include but are not limited to N-(2,3-ditetradecanoyloxy propyl)-N,N,N-trimethyl ammonium chloride (DOTAP), N-(2,3-dioleyloxy propyl)-N,N,N-trimethyl ammonium chloride (DOTMA), distearoyl phosphatidylcholine (DSPC), dioleoyl phosphatidylcholine (DOPC), dipalmitoyl phosphatidylcholine (DPPC), dioleoyl phosphatidylglycerol (DOPG), dioleoyl phosphatidylethanolamine (DOPE), 3β-[N-(N',N'-dimethylaminoethane)-carbamoyl] cholesterol (DC-chol), dimyristoyl phosphatidylcholine (DMPC), dimyristoyl phosphatidylethanolamine (DMPE), dioleoyl dimethylammonium chloride (DODAC), 1-palmitoyl-2-oleoyl-sn-glycero-3-phosphocholine (POPC), 1,2-dioleoyl-3-trimethylammonium-propane (DOTAP-CI), 1,2-dioleyl-3-dimethylaminopropane (DODMA), 1,2-dimyristoyl-rac-glycerol methoxy polyethylene glycol (DMG-PEG2000), 2-[(spermine carbamoylamino)ethyl]-N,N-dimethyl-3-amino propane trifluoroacetate (DOSPA), 1,2-dioleoyl-3-dimethylammonium-propane (DODAP), 2,3-dioleoyloxy-N-[2-(spermine carboxamido)ethyl]-N,N-dimethyl-1-propanaminium trifluoroacetate (DOSPA), 28-carbamoylamidospermine carbamoyl glutamate (DOGS), dioleoyl ethylphosphatidylcholine (DOEPC), diphphytanoyl phosphatidylethanolamine (DPhPE), dipalmitoyl phosphatidylethanolamine (DPPE), 3β-[1-(aminoiminomethyl)amino] cholesterol (O-Chol), palmitoyloleoyl phosphatidylethanolamine (POPE), dipalmitoyloleoyl phosphatidylglycerol (DPPG) and other commercialized liposomes, or any mixture of the aforementioned liposomes.

Preferably, the cyclic peptide or cyclic peptide and drug composition can form complex particles with one or more of the specified liposomes.

On the other hand, the present invention provides that any of the aforementioned cyclic peptides or cyclic peptide compositions can be used in combination with one or more pharmaceutically acceptable carriers, buffers, diluents, and excipients.

The carrier, chosen according to the route of application, can be in solid or liquid form. Routes of application include, but are not limited to, dermal or transdermal, oral, parenteral, including subcutaneous, intramuscular, or intravenous injection, or regional routes such as topical or intranasal. Suitable quantities of the compositions (drugs) pursuant to the present invention can be determined through routine experimentation with animal models, which include humans and animals and not limited to pet species such as cats or dogs, farm animals including but not limited to cows, horses, goats, sheep, and pigs, research animals such as mice, rats, rabbits, goats, sheep, pigs, dogs, and cats, avians such as chickens, turkeys, and passerines, and groups consisting of humans, goats, cows, pigs, dogs, cats, monkeys, apes, or rodents, inclusive of mice, hamsters, and rabbits.

The cyclic peptide structures or cyclic peptide and drug compositions are for use in preparations aimed at preventing, treating, and/or improving a selection of diseases from the following: cancer or tumorous conditions, viral or bacterial infections, genetic disorders, respiratory system diseases, cardiovascular diseases, neurological disorders, digestive system diseases, skeletal diseases, connective tissue diseases, immunodeficiency diseases, endocrine diseases, and diseases of the eyes and ears.

The cyclic peptide structures or cyclic peptide and drug compositions may also be used for the prevention, treatment, and/or improvement of a selection of animal diseases, intended for veterinary drug compositions, or as animal vaccines.

Preferably, the novel cyclic peptides of the present invention can select from, but are not limited to, a subset of the following general formulas, including one or more combinations thereof, with the amino acid sequences as shown in Table 1, where a disulfide bond is formed between two cysteines (Cys):

**Table 1**

| Cyclic peptide name | Serial number | Amino acid sequence |
|---|---|---|
| CP3-19-A-1 | SEQ ID No.1 | Ala-Ala-Leu-Ala-Ala-Leu-Cys-Lys-Lys-Lys-Lys-Lys-Cys- Leu-Ala-Ala-Leu-Ala-Ala |
| CP3-25-A-1 | SEQ ID No.2 | Ala-Ala-Leu-Ala-Ala-Leu-Ala-Ala-Leu-Cys-Lys-Lys-Lys- |
| | | Lys-Lys-Cys-Leu-Ala-Ala-Leu-Ala-Ala-Leu-Ala-Ala |
| CP3-25-A-2 | SEQ ID No.3 | Lys-Ala-Leu-Lys-Ala-Leu-Lys-Ala-Leu-Cys-Ala-Leu-Lys- Leu-Ala-Cys-Leu-Ala-Lys-Leu-Ala-Lys-Leu-Ala-Lys |
| CP4-25-A-1 | SEQ ID No.4 | Ala-Lys-Ala-Leu-Ala-Lys-Ala-Leu-Cys-Lys-Lys-Lys-Lys- Lys-Lys-Lys-Cys-Leu-Ala-Lys-Ala-Leu-Ala-Lys-Ala |
| CP4-25-AQ-1 | SEQ ID No.5 | Ala-Lys-Ala-Leu-Ala-Lys-Ala-Leu-Cys-Gln-Lys-Lys-Lys- Lys-Lys-Gln-Cys-Leu-Ala-Lys-Ala-Leu-Ala-Lys-Ala |
| CP4-25-AQ-2 | SEQ ID No.6 | Ala-Ala-Ala-Leu-Ala-Ala-Ala-Leu-Cys-Gln-Lys-Lys-Lys- Lys-Lys-Gln-Cys-Leu-Ala-Ala-Ala-Leu-Ala-Ala-Ala |
| CP4-25-MQ-1 | SEQ ID No.7 | Lys-Lys-Met-Leu-Lys-Lys-Met-Leu-Cys-Gln-Lys-Lys-Lys- Lys-Lys-Gln-Cys-Leu-Met-Lys-Lys-Leu-Met-Lys-Lys |
| CP4-25-A-2 | SEQ ID No.8 | Lys-Lys-Lys-Leu-Lys-Lys-Lys-Leu-Cys-Ala-Leu-Ala-Lys- Ala-Leu-Ala-Cys-Leu-Lys-Lys-Lys-Leu-Lys-Lys-Lys |
| CP3-28-Q-1 | SEQ ID No.9 | Lys-Gln-Leu-Lys-Gln-Leu-Cys-Lys-Lys-Lys-Lys-Lys-Lys- Lys-Lys-Lys-Lys-Lys-Lys-Lys-Lys-Cys-Leu-Gln-Lys-Leu- Gln-Lys |
| CP3-28-Q-2 | SEQ ID No.10 | Lys-Gln-Leu-Lys-Gln-Leu-Cys-Gln-Gln-Lys-Gln-Gln-Lys- Gln-Gln-Lys-Gln-Gln-Lys-Gln-Gln-Cys-Leu-Gln-Lys-Leu- Gln-Lys |
| CP3-28-1 | SEQ ID No.11 | Lys-Lys-Leu-Lys-Lys-Leu-Cys-Lys-Lys-Lys-Lys-Lys-Lys- Lys-Lys-Lys-Lys-Lys-Lys-Lys-Lys-Cys-Leu-Lys-Lys-Leu- Lys-Lys |
| CP3-28-2 | SEQ ID No.12 | Lys-Leu-Leu-Lys-Leu-Leu-Cys-Lys-Lys-Lys-Lys-Lys-Lys- Lys-Lys-Lys-Lys-Lys-Lys-Lys-Lys-Cys-Leu-Leu-Lys-Leu- Leu-Lys |
| CP3-28-3 | SEQ ID No.13 | Lys-Leu-Leu-Lys-Leu-Leu-Cys-Leu-Lys-Lys-Leu-Lys- Lys-Leu-Lys-Lys-Leu-Lys-Lys-Lys-Leu-Cys-Leu-Leu- Lys-Leu-Leu-Lys |
| CP3-28-A-1 | SEQ ID No.14 | Lys-Lys-Leu-Lys-Lys-Leu-Cys-Ala-Lys-Lys-Ala-Lys-Lys- Ala-Lys-Lys-Ala-Lys-Lys-Lys-Ala-Cys-Leu-Leu-Lys-Leu- Leu-Lys |
| CP3-34-A-1 | SEQ ID No.15 | Lys-Lys-Leu-Lys-Lys-Leu-Lys-Lys-Leu-Cys-Ala-Lys-Lys- Ala-Lys-Lys-Ala-Lys-Lys-Ala-Lys-Lys-Lys-Ala-Cys-Leu- Leu-Lys-Leu-Leu-Lys-Leu-Leu-Lys |
| CP3-34-AF-1 | SEQ ID No.16 | Lys-Lys-Leu-Lys-Lys-Leu-Lys-Lys-Leu-Cys-Ala-Phe-Lys- Ala-Phe-Lys-Ala-Phe-Lys-Ala-Phe-Lys-Phe-Ala-Cys- Leu-Leu-Lys-Leu-Leu-Lys-Leu-Leu-Lys |
| CP3-28-AF-1 | SEQ ID No.17 | Lys-Lys-Leu-Lys-Lys-Leu-Lys-Lys-Leu-Lys-Lys-Leu-Cys- Ala-Phe-Lys-Ala-Phe-Lys-Ala-Phe-Lys-Ala-Phe-Lys-Phe- Ala-Cys |
| CP3-25-AF-1 | SEQ ID No.18 | Ala-Lys-Leu-Ala-Lys-Leu-Ala-Lys-Leu-Cys-Ala-Phe-Lys- Ala-Phe-Lys-Ala-Phe-Lys-Ala-Phe-Lys-Phe-Ala-Cys |
| CP4-33-AM-1 | SEQ ID No.19 | Lys-Lys-Lys-Leu-Lys-Lys-Lys-Leu-Cys-Ala-Leu-Ala-Lys- Ala-Met-Ala-Lys-Ala-Met-Ala-Lys-Ala-Met-Ala-Cys-Leu- Lys-Lys-Lys-Leu-Lys-Lys-Lys |
| CP4-33-AF-1 | SEQ ID No.20 | Lys-Lys-Lys-Leu-Lys-Lys-Lys-Leu-Cys-Ala-Phe-Ala-Lys- Ala-Phe-Ala-Lys-Ala-Phe-Ala-Lys-Ala-Phe-Ala-Cys-Leu- |
| | | Lys-Lys-Lys-Leu-Lys-Lys-Lys |
| CP4-37-QW-1 | SEQ ID No.21 | Lys-Leu-Leu-Lys-Leu-Leu-Lys-Leu-Leu-Lys-Leu-Leu- Cys-Lys-Lys-Lys-Lys-Lys-Cys-Leu-Leu-Lys-Leu-Leu- Lys-Leu-Leu-Lys-Leu-Leu-Lys |
| CP4-37-QF-1 | SEQ ID No.22 | Lys-Lys-Lys-Leu-Lys-Lys-Lys-Leu-Cys-Phe-Gln-Phe- Lys-Phe-Gln-Phe-Lys-Phe-Gln-Phe-Lys-Phe-Gln-Phe- Lys-Phe-Gln-Phe-Cys-Leu-Lys-Lys-Lys-Leu-Lys-Lys-Lys |
| CP4-37-QV-1 | SEQ ID No.23 | Lys-Gln-Val-Leu-Lys-Gln-Val-Leu-Cys-Gln-Lys-Lys-Lys- Gln-Lys-Lys-Lys-Gln-Lys-Lys-Lys-Gln-Lys-Lys-Lys-Lys- Lys-Gln-Cys-Leu-Val-Gln-Lys-Leu-Val-Gln-Lys |
| CP4-41-AM-1 | SEQ ID No.24 | Lys-Ala-Met-Leu-Lys-Ala-Met-Leu-Lys-Ala-Met-Leu-Cys- Lys-Lys-Lys-Lys-Lys-Lys-Lys-Lys-Lys-Lys-Lys-Lys-Lys- Lys-Lys-Cys-Leu-Met-Ala-Lys-Leu-Met-Ala-Lys-Leu-Met- Ala-Lys |
| CP4-41-AF-1 | SEQ ID No.25 | Lys-Ala-Phe-Leu-Lys-Ala-Phe-Leu-Lys-Ala-Phe-Leu- Cys-Lys-Lys-Lys-Lys-Lys-Lys-Lys-Lys-Lys-Lys-Lys-Lys- Lys-Lys-Lys-Cys-Leu-Phe-Ala-Lys-Leu-Phe-Ala-Lys- Leu-Phe-Ala-Lys |
| CP4-41-AQ-1 | SEQ ID No.26 | Ala-Lys-Ala-Leu-Ala-Lys-Ala-Leu-Ala-Lys-Ala-Leu-Cys- Gln-Lys-Lys-Lys-Gln-Lys-Lys-Lys-Gln-Lys-Lys-Lys-Gln- Lys-Lys-Cys-Leu-Ala-Lys-Ala-Leu-Ala-Lys-Ala-Leu-Ala- Lys-Ala |
| CP4-41-AQ-2 | SEQ ID No.27 | Ala-Lys-Ala-Leu-Ala-Lys-Ala-Leu-Ala-Lys-Ala-Leu-Ala- Lys-Ala-Leu-Ala-Lys-Ala-Leu-Ala-Lys-Ala-Leu-Cys-Gln- Lys-Lys-Lys-Gln-Lys-Lys-Lys-Gln-Lys-Lys-Lys-Lys-Lys- Gln-Cys |
| CP4-43-QM-1-RDG | SEQ ID No.28 | Arg-Asp-Gly-Lys-Lys-Lys-Leu-Lys-Lys-Lys-Leu-Cys-Gln- Leu-Met-Lys-Gln-Leu-Met-Lys-Gln-Leu-Met-Lys-Gln- Leu-Met-Lys-Met-Leu-Gln-Cys-Leu-Lys-Lys-Lys-Leu- Lys-Lys-Lys-Gly-Asp-Arg |
| CP4-43-QI-1-RDG | SEQ ID No.29 | Arg-Asp-Gly-Lys-Lys-Lys-Leu-Lys-Lys-Lys-Leu-Cys-Gln- Leu-Ile-Lys-Gln-Leu-Ile-Lys-Gln-Leu-Ile-Lys-Gln-Leu-Ile- Lys-Ile-Leu-Gln-Cys-Leu-Lys-Lys-Lys-Leu-Lys-Lys-Lys- Gly-Asp-Arg |
| CP4-43-VQ-1-RDG | SEQ ID No.30 | Arg-Asp-Gly-Lys-Val-Gln-Leu-Lys-Val-Gln-Leu-Cys-Gln- Lys-Lys-Lys-Gln-Lys-Lys-Lys-Gln-Lys-Lys-Lys-Gln-Lys- Lys-Lys-Lys-Lys-Gln-Cys-Leu-Gln-Val-Lys-Leu-Gln-Val- Lys-Gly-Asp-Arg |
| CP4-40-QW-1-RDG | SEQ ID No.31 | Arg-Asp-Gly-Lys-Lys-Lys-Leu-Lys-Lys-Lys-Leu-Lys-Lys- Lys-        Leu-Lys-Lys-Lys-Leu-Cys-Gln-Leu-Trp-Lys-Gln- Leu-Trp-Lys-Gln-Leu-Trp-Lys-Gln-Leu-Trp-Lys-Trp-Leu- Gln-Cys |

The technical solution of the cyclic peptide or cyclic peptide compositions described in this invention provides a novel cyclic peptide carrier with high gene delivery efficiency, structural stability, and favorable biocompatibility, including its variant forms. The technical solution of this invention enables efficient delivery of nucleic acids to tissues and organs within the body for targeted gene regulation therapy. Compared to existing delivery carriers, the present invention offers a series of beneficial technical effects.

The cyclic peptides or cyclic peptide compositions of this invention form a relatively tight bond with nucleic acid drugs, creating uniform and stable nanoparticles which can be preserved in liquid or solid form, allowing for flexible administration methods. In addition to conventional intravenous injection, other administration methods such as intramuscular injection, nebulization, powder, or oral dosage forms can also be employed.

The cyclic peptides or cyclic peptide compositions described by this invention are composed of commonly found amino acids in the human body, and the allergic reactions are generally mild. Usually, there is no need for antihistamines and steroid medication before injection, which makes the drugs suitable not only for rare diseases and serious illnesses such as cancer but also for infectious diseases.

The manufacture of the cyclic peptides or cyclic peptide compositions described in this invention is relatively controllable. Additionally, the biodegradability and safety of these cyclic peptide compositions are favorable, with low side effects and an enhanced therapeutic index. The biological response of these compositions is favorable-they can effectively carry nucleic acid drugs into the cells within the body's tissues and organs, where they release the nucleic acids through endosomal escape to elicit a therapeutic effect.

The present invention describes cyclic peptides or cyclic peptide compositions that employ the orderly arrangement of amino acid sequences and disulfide bond coupling into a ring to effectively enhance the robustness of peptide molecular structure, transmembrane activity, and metabolic stability. Thus, it provides an optimized technical solution for molecular medical therapies that safely and efficiently introduce specific or regular sequences of nucleic acids into the cells or tissues of patients or animals.

### BRIEF DESCRIPTION OF THE DRAWINGS

The accompanying drawings serve to further illustrate the present invention and should not be construed as limiting the subject matter of the invention.
FIG. 1 shows the results of agarose gel electrophoresis of a cyclic peptide with siRNA at different mass ratios.
FIG. 2 shows the results of agarose gel electrophoresis of another cyclic peptide with siRNA at different weight ratios.
FIG. 3 is a schematic illustration demonstrating that cyclic peptide S5 can carry mRNA into 293T cells and express the target fluorescent protein.
FIG. 4 presents a chromatogram illustrating the particle size analysis of cyclic peptide/lipid/mRNA compound preparations (CLS) and lipid nanoparticle (LNP) samples.
FIG. 5 is a schematic demonstrating that the cyclic peptide/lipid/mRNA compound construction (CLS) can efficiently carry mRNA into various cell lines and express the target fluorescent protein.
FIG. 6 illustrates that the cyclic peptide/lipid/siRNA compound preparation can deliver siRNA effectively in mice via intravenous injection and knock down the expression of the target gene.
FIG. 7 illustrates that the cyclic peptide/lipid/siRNA compound preparation can deliver siRNA effectively in mice via intramuscular injection and knock down the expression of the target gene.
FIG. 8 shows a transmission electron microscope image of nanoparticles formed from the cyclic peptide/lipid/siRNA compound preparation.
FIG. 9 shows that the cyclic peptide/lipid/mRNA compound formulation (CLS) can effectively deliver mRNA in mice and express the target gene protein.
FIG. 10 shows that the cyclic peptide/lipid/mRNA compound formulation (CLS) can effectively carry mRNA and express the target gene protein within mice.
FIG. 11 is a mass spectrum of cyclic peptides prepared in Example 9.
FIG. 12 is a liquid chromatography diagram of cyclic peptides prepared in Example 9.
FIG. 13 shows the fluorescence expression values for further screening of CLS formulations efficiently carrying mRNA into 293T cells in Example 12.
FIG. 14 displays the fluorescence expression values in 293T cells transfected with CLS formulation in Example 15.
FIG. 15 is a gel electrophoresis encapsulation chart of the CLS formulation in Example 16.
FIG. 16 illustrates the effective delivery of mRNA within mice by intravenous injection of the CLS formulation and the expression of the target gene protein in Example 17.
FIG. 17 shows the values of the effective delivery of mRNA within mice by intravenous injection of the CLS formulation and the expression of the target gene protein in Example 17.
FIG. 18 represents the expression of the target fluorescent protein in mice given the CLS formulation by muscle injection in Example 18.
FIG. 19 shows the expression values of the target fluorescent protein in mice given the CLS formulation by muscle injection in Example 18.

### DETAILED DESCRIPTION OF THE PREFERRED

### EMBODIMENTS

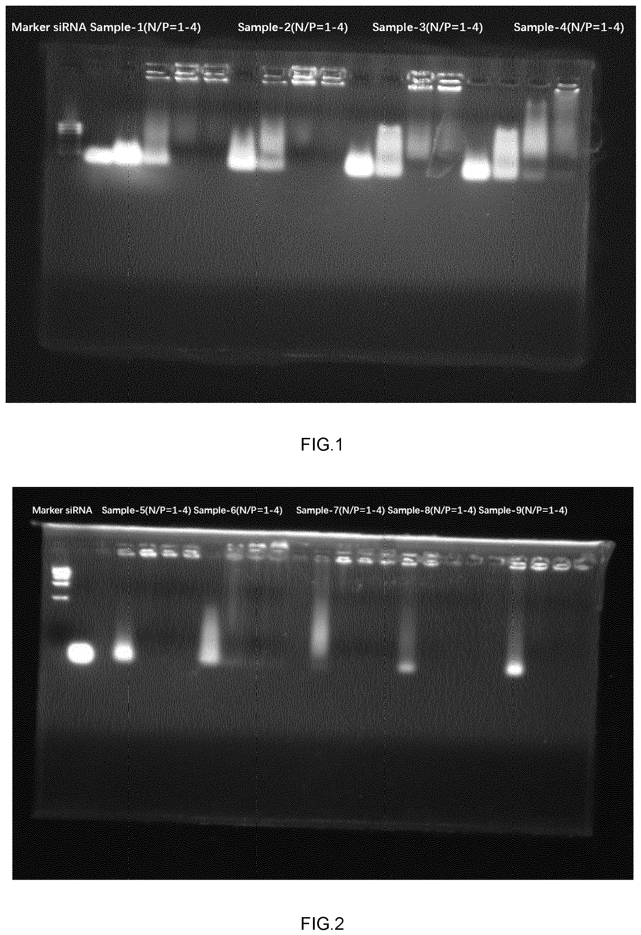

The following sections, combined with specific examples and Figures 1-19, provide further detailed explanations of the present invention, allowing those skilled in the art to better understand it, thereby enabling a clearer and more precise definition of the scope of protection of the invention.

Below are the descriptions for Figures 1-10, which will also be further explained in conjunction with the drawings in specific examples.

### Part One

In Figure 1, the bands from left to right sequentially are: Sample 1/siRNA mass ratios 1-4; Sample 2/siRNA mass ratios 1-4; Sample 3/siRNA mass ratios 1-4; Sample 4/siRNA mass ratios 1-4. In Figure 2, the bands from left to right are sequentially: Sample 5/siRNA mass ratios 1-4; Sample 6/siRNA mass ratios 1-4; Sample 7/siRNA mass ratios 1-4; Sample 8/siRNA mass ratios 1-4; Sample 9/siRNA mass ratios 1-4. Among them, Sample-1(N/P=1-4) indicates Sample 1 with siRNA mass ratio of 1-4; Sample-2(N/P=1-4) indicates Sample 2 with siRNA mass ratio of 1-4; Sample-3(N/P=1-4) indicates Sample 3 with siRNA mass ratio of 1-4; Sample-4(N/P=1-4) indicates Sample 4 with siRNA mass ratio of 1-4; Sample-5(N/P=1-4) indicates Sample 5 with siRNA mass ratio of 1-4; Sample-6(N/P=1-4) indicates Sample 6 with siRNA mass ratio of 1-4; Sample-7(N/P=1-4) indicates Sample 7 with siRNA mass ratio of 1-4; Sample-8(N/P=1-4) indicates Sample 8 with siRNA mass ratio of 1-4; Sample-9(N/P=1-4) indicates Sample 9 with siRNA mass ratio of 1-4. The agarose gel electrophoresis experiments show that Samples 1-3 and Sample 6 can encapsulate siRNA well at a mass ratio of 4:1 of cyclic peptide/siRNA. The encapsulation by Sample 4 is poor, with some siRNA remaining unencapsulated even at a mass ratio of 4:1. Samples 5, 7, 8, and 9 also encapsulate siRNA well at a mass ratio of 4:1. This demonstrates that cyclic peptides can effectively bind and carry nucleic acids.

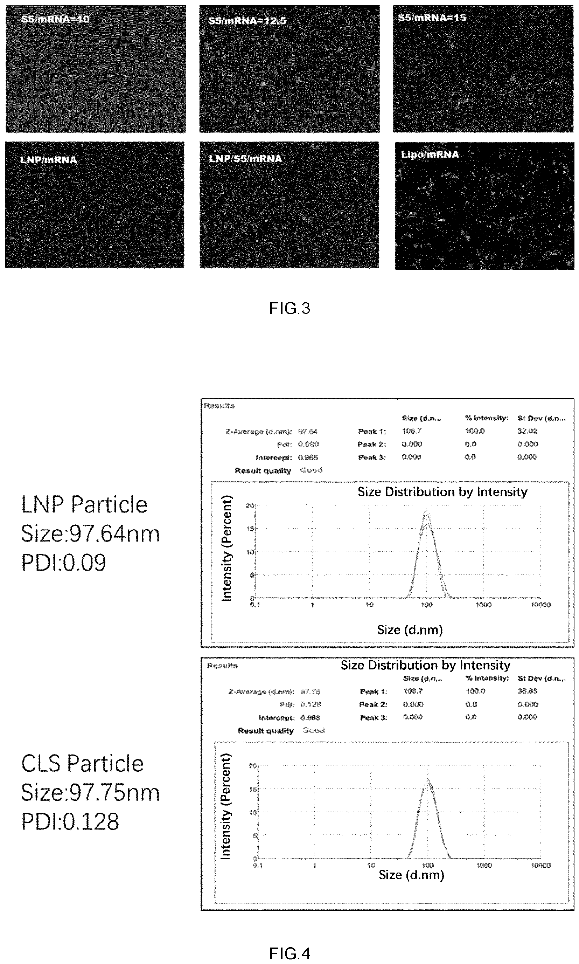

Figure 3 demonstrates that cyclic peptide S5 can carry mRNA into 293T cells and express the target fluorescent protein, with an increase in fluorescence intensity corresponding to the increase in the proportion of cyclic peptide. The cyclic peptide/lipid/mRNA compound formulation (CLS) also showed higher fluorescence expression intensity compared to the control group LNP. This indicates that cyclic peptides can effectively carry nucleic acids into cells. Where S5/mRNA=10 denotes a sample 5/mRNA mass ratio of 10:1, S5/mRNA=12.5 indicates a sample 5/mRNA mass ratio of 12.5:1, S5/mRNA=15 signifies a sample 5/mRNA mass ratio of 15:1, LNP/mRNA indicates lipid nanoparticles carrying mRNA, LNP/S5/mRNA signifies lipid nanoparticles mixed with sample 5 carrying mRNA, and Lipo/mRNA represents the commercial reagent for transfecting mRNA.

Figure 4 presents a chromatogram showing particle size analysis for cyclic peptide/lipid/mRNA compound preparation (CLS) and control group lipid nanoparticle (LNP) samples, with both groups averaging about 100nm in particle size and a polydispersity index (PDI) less than 0.2, suggesting that cyclic peptides can form uniformly sized nanoparticles when combined with nucleic acids. Here, LNP denotes lipid nanoparticles, CLS indicates cyclic peptide/lipid/mRNA compound preparation, and PDI is the polydispersity index, reflecting the uniformity of particle size distribution. Based on the PDI results, samples 3, 5, and 7-9 showed better outcomes (less than 0.3). After comprehensive analysis, samples 5 and 7-9 had the best particle size results. Consequently, samples 5 and 8 were selected for dynamic light scattering analysis, which showed that their average particle size increased after binding with lipids, but both were within normal particle size ranges, and the PDI was also satisfactory. Therefore, sample 5 was chosen for in vitro fluorescent transfection experiments and animal studies.

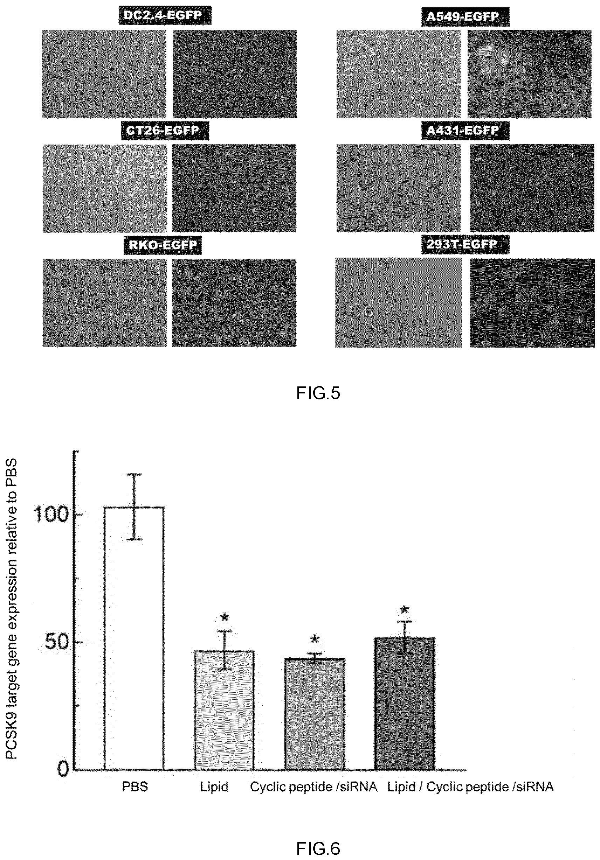

Figure 5 shows that cyclic peptide/lipid/mRNA compound formulation (CLS) can effectively carry mRNA and transfect various cell lines, expressing the target fluorescent protein. Included among these, DC2.4 are dendritic cells, A549 are human non-small cell lung cancer cells, CT26 are mouse colon cancer cells, A431 are human epidermoid carcinoma cells, RKO are human colon cancer cells, and 293T are human renal epithelial cells, with EGFP representing green fluorescent protein.

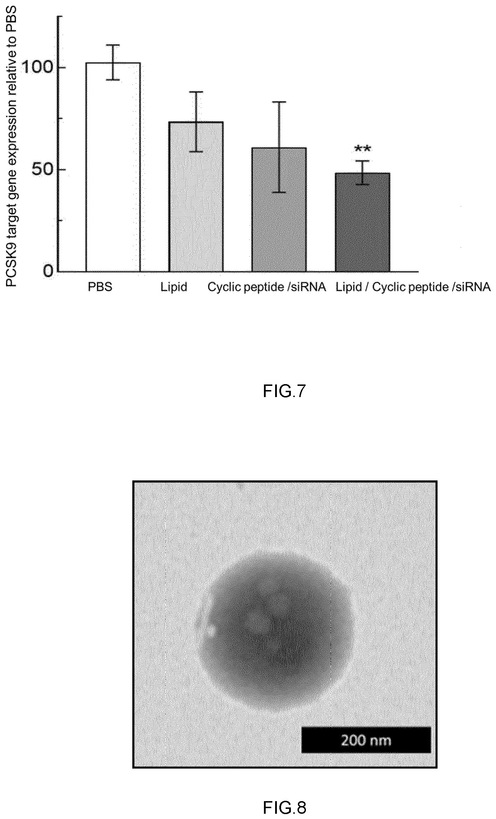

Figures 6 and 7 show that cyclic peptide/lipid/siRNA compound formulations can effectively deliver siRNA inside mice using intravenous and intramuscular administration, consequently knocking down the expression of the target gene.

Figure 8 displays a transmission electron microscope image of the nanoparticles formed by the cyclic peptide/lipid/siRNA compound preparation.

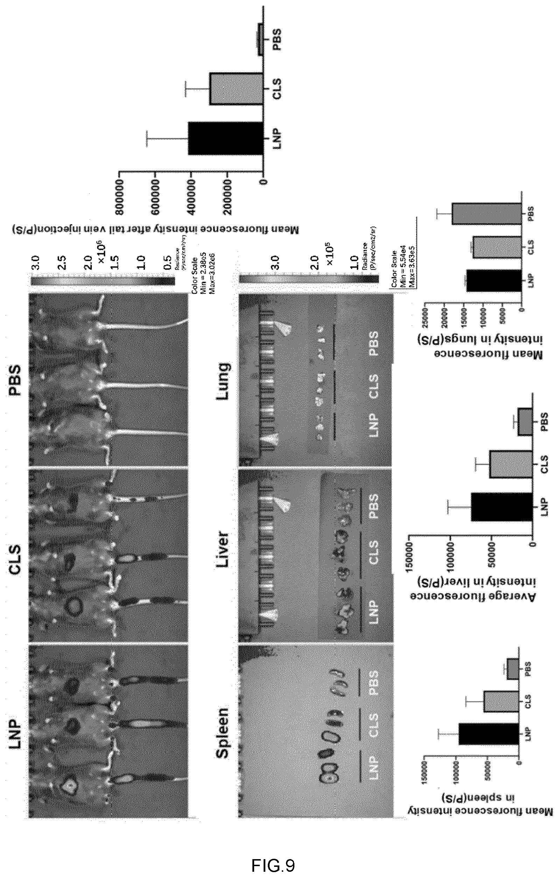

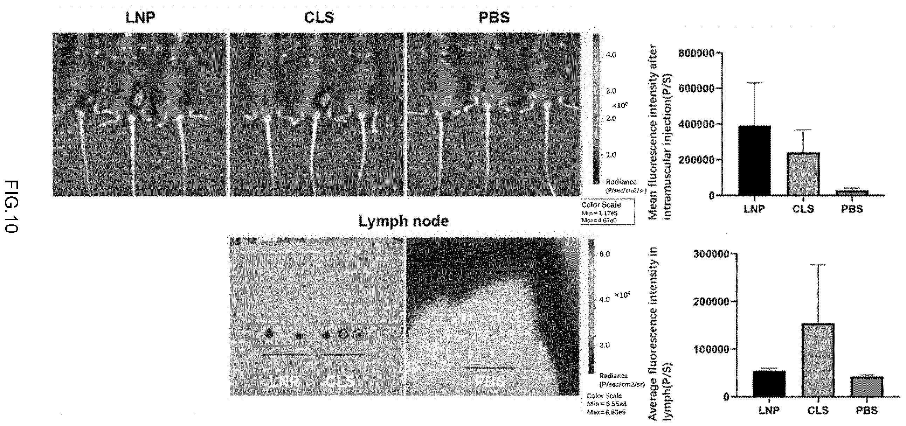

Figures 9 and 10 demonstrate that cyclic peptide/lipid/mRNA compound formulations (CLS) can effectively deliver mRNA in mice and express the target gene protein. Here, CLS denotes cyclic peptide/lipid/mRNAcompound formulations, and LNP signifies lipid nanoparticles.

The cyclic peptides used in Examples 1-8 are presented in the following Table 2:

**Table 2**

| Cyclic peptide Experimen t No. | Serial number | Amino acid sequence |
|---|---|---|
| Sample 1 | SEQ ID No.2 | Ala-Ala-Leu-Ala-Ala-Leu-Ala-Ala-Leu-Cys-Lys-Lys- Lys-Lys-Lys-Cys-Leu-Ala-Ala-Leu-Ala-Ala-Leu-Ala- Ala |
| Sample 2 | SEQ ID No.7 | Lys-Lys-Met-Leu-Lys-Lys-Met-Leu-Cys-Gln-Lys-Lys- Lys-Lys-Lys-Gln-Cys-Leu-Met-Lys-Lys-Leu-Met-Lys- Lys |
| Sample 3 | SEQ ID No.11 | Lys-Lys-Leu-Lys-Lys-Leu-Cys-Lys-Lys-Lys-Lys-Lys- Lys-Lys-Lys-Lys-Lys-Lys-Lys-Lys-Lys-Cys-Leu-Lys- Lys-Leu-Lys-Lys |
| Sample 4 | SEQ ID No.17 | Lys-Lys-Leu-Lys-Lys-Leu-Lys-Lys-Leu-Lys-Lys-Leu- Cys-Ala-Phe-Lys-Ala-Phe-Lys-Ala-Phe-Lys-Ala-Phe- Lys-Phe-Ala-Cys |
| Sample 5 | SEQ ID No.21 | Lys-Leu-Leu-Lys-Leu-Leu-Lys-Leu-Leu-Lys-Leu- Leu-Cys-Lys-Lys-Lys-Lys-Lys-Cys-Leu-Leu-Lys-Leu- Leu-Lys-Leu-Leu-Lys-Leu-Leu-Lys |
| Sample 6 | SEQ ID No.23 | Lys-Gln-Val-Leu-Lys-Gln-Val-Leu-Cys-Gln-Lys-Lys- Lys-Gln-Lys-Lys-Lys-Gln-Lys-Lys-Lys-Gln-Lys-Lys- Lys-Lys-Lys-Gln-Cys-Leu-Val-Gln-Lys-Leu-Val-Gln- |
| | | Lys |
| Sample 7 | SEQ ID No.24 | Lys-Ala-Met-Leu-Lys-Ala-Met-Leu-Lys-Ala-Met-Leu- Cys-Lys-Lys-Lys-Lys-Lys-Lys-Lys-Lys-Lys-Lys-Lys- Lys-Lys-Lys-Lys-Cys-Leu-Met-Ala-Lys-Leu-Met-Ala- Lys-Leu-Met-Ala-Lys |
| Sample 8 | SEQ ID No.29 | Arg-Asp-Gly-Lys-Lys-Lys-Leu-Lys-Lys-Lys-Leu-Cys- Gln-Leu-Ile-Lys-Gln-Leu-Ile-Lys-Gln-Leu-Ile-Lys-Gln- Leu-Ile-Lys-Ile-Leu-Gln-Cys-Leu-Lys-Lys-Lys-Leu- Lys-Lys-Lys-Gly-Asp-Arg |
| Sample 9 | SEQ ID No.30 | Arg-Asp-Gly-Lys-Val-Gln-Leu-Lys-Val-Gln-Leu-Cys- Gln-Lys-Lys-Lys-Gln-Lys-Lys-Lys-Gln-Lys-Lys-Lys- Gln-Lys-Lys-Lys-Lys-Lys-Gln-Cys-Leu-Gln-Val-Lys- Leu-Gln-Val-Lys-Gly-Asp-Arg |

Peptide chains were synthesized by sequentially coupling the protected amino acids from the C-terminus using solid-phase synthesis, with an excess of protected amino acids and coupling reagents to ensure completeness of each reaction step. After formation of the disulfide bond between the two cysteines, the protecting groups and the peptide resin were cleaved in a trifluoroacetic acid solution. Precipitation with methyl tert-butyl ether was then carried out, followed by centrifugation and drying to obtain crude product which was subsequently purified by preparative high-performance liquid chromatography to yield the target peptide. The synthesis was performed by Hangzhou Zhong peptide Biochemical Co., Ltd. and Shanghai Ji'er Peptide Co., Ltd.

### Embodiment 1: Agarose Gel Electrophoresis Experiment.

a. Weigh out 0.5g of agarose powder and pour it into a conical flask, then add 50 mL of 1xTAE electrophoresis buffer into the flask. Dissolve it completely under heating conditions.
b. After cooling to 65°C, add 5 µL of ethidium bromide, swiftly pour into the gel casting tray, insert the comb, and let it stand at room temperature for 0.5-1 hour until the gel solidifies.
c. Next, remove the comb, pour 1×TAE electrophoresis buffer into the well to submerge the gel.
d. Prepare nine formulations of cyclic peptides with siRNA, using samples 1-9 at mass ratios of 1-4 of sample/siRNA. Mix 2 µL of 6× loading dye with 10 µL of samples, load 10 µL into the wells, and concurrently load 10 µL of DNA Marker and bare siRNA into the wells as controls.
e. Subsequently, run the electrophoresis at 110mV for 45 minutes, turn off the power supply, remove the gel, and observe and record the electrophoresis image with a UV gel imaging system.
f. Results are shown in Figures 1 and 2, samples 1-3 and sample 6 were able to adequately encapsulate siRNA at a mass ratio of 4:1 of cyclic peptide/siRNA. Sample 4 showed poor encapsulation effects, with some siRNA still not encapsulated at a mass ratio of 4:1. Samples 5, 7, 8, and 9 were able to encapsulate siRNA effectively at a mass ratio of 4:1.

Figure 1 bands from left to right are as follows: sample 1/siRNA mass ratios of 1-4; sample 2/siRNA mass ratios of 1-4; sample 3/siRNA mass ratios of 1-4; sample 4/siRNA mass ratios of 1-4. Figure 2 bands from left to right are as follows: sample 5/siRNA mass ratios of 1-4; sample 6/siRNA mass ratios of 1-4; sample 7/siRNA mass ratios of 1-4; sample 8/siRNA mass ratios of 1-4; sample 9/siRNA mass ratios of 1-4. This indicates that cyclic peptides can effectively bind and carry nucleic acids.

### Embodiment 2: Particle Size Measurement Experiment.

Utilizing the Malvern Zetasizer Nano ZS (Malvern Nano Particle Sizing Instrument), the particle size (Size), polydispersity index (PDI), and zeta potential (Zeta P) of the particles formed after samples 1-9 encapsulate siRNA at a cyclic peptide/RNA mass ratio of 4:1 were measured by dynamic light scattering (DLS).

The measurement data are shown in Table 3.

**Table 3**

| Experiment No. | Size (nm) | Polydispersity index | Zeta potential |
|---|---|---|---|
| 1 | 159.6 | 0.254 | 7.09 |
| 2 | 211.9 | 0.326 | 34.1 |
| 3 | 299.1 | 0.065 | 21.3 |
| 4 | 264.5 | 0.542 | 18.6 |
| 5 | 81.02 | 0.133 | 81.02 |
| 6 | 32.58 | 0.355 | 21.2 |
| 7 | 145.1 | 0.139 | 26.3 |
| 8 | 29.92 | 0.143 | 16.5 |
| 9 | 111.3 | 0.169 | 23.0 |

In this experiment, sample number 1 corresponds to the formulation of sample 1 with siRNA, sample number 2 corresponds to the formulation of sample 2 with siRNA, and so forth, up to sample number 9 corresponding to the formulation of sample 9 with siRNA.

Based on the dynamic light scattering measurements, the particle size of the formulations for samples 2-4 were relatively large (greater than 200nm), while the formulations for samples 1 and 5-9 exhibited more desirable particle sizes. When considering the polydispersity index (PDI) results, the formulations for samples 3, 5, and 7-9 were favorable (less than 0.3). Upon comprehensive analysis, the formulations for samples 5 and 7-9 demonstrated the most optimal particle size results.

### Embodiment 3: Particle Size Measurement of Cyclic Peptide/Lipid/siRNA Formulations.

Sample 5 and 8 were selected and initially mixed in a microfluidic channel at a mass ratio of 3:1 cyclic peptide/RNA to form a particle solution, which was then left to stabilize.

The mass ratio of distearoylphosphatidylcholine (DSPC)/cholesterol/RNA was set at 2.4/3.1/1. DSPC and cholesterol were weighed and dissolved in a certain volume of ethanol solution.

The cyclic peptide/siRNA aqueous solution was mixed with the lipid ethanol solution through a microfluidic channel to form composite particles again, where the final concentration of ethanol was 25%. Dialysis was used to remove the ethanol, and the final lipid/cyclic peptide/nucleic acid composite particles were collected.

Using the Malvern Zetasizer Nano ZS, the particle size and polydispersity index (PDI) before and after binding with lipids (5-L and 8-L) were measured for sample 5 and 8 using dynamic light scattering.

| Sample | Size (nm) | PDI |
|---|---|---|
| 5 | 60.17 | 0.176 |
| 5-L | 145.1 | 0.139 |
| 8 | 31.85 | 0.15 |
| 8-L | 114.80 | 0.09 |

In this context, 5 represents cyclic peptide sample 5/siRNA formulation, 5-L represents cyclic peptide sample 5/lipid/siRNA formulation, 8 represents cyclic peptide sample 8/siRNA formulation, and 8-L represents cyclic peptide sample 8/lipid/siRNA formulation.

Based on the dynamic light scattering measurements, the average particle size (Size) of samples 5 and 8 increased after lipid binding, but both were within the normal particle size range, and the polydispersity index (PDI) was also satisfactory.

### Embodiment 4: Testing of Cyclic Peptide/Lipid/mRNA (CLS) Formulations.

The preparation of cyclic peptide/lipid/mRNA (EGFP green fluorescent protein) compound formulation was conducted as follows:
1. Sample preparation: Sample 5 (S5) was selected and mixed in a microfluidic channel to form a particle solution at different cyclic peptide/mRNA mass ratios (10:1, 12.5:1, 15:1), and then left to stabilize.
2. Control formulation: A lipid nanoparticle (LNP) control at a ratio of 4-(N,N-dimethylamino) butyric acid (dilinoleyl) methyl ester/DSPC/cholesterol/1,2-dimyristoyl-rac-glycerol-3-methoxypolyethylene glycol 2000 = 50:10:38.5:1.5 was used and mRNA was encapsulated according to the method described in Embodiment 3.
3. Cell plating: 293T cells were plated in a 24-well plate with 9 wells per cell type, each well containing 1ml of medium and 1×10^5 cells, grown for 12 hours until adherence.
4. Solution preparation: PBS (control); for each group in 3ml serum-free medium, 3×20µl = 60µl of prepared sample was added and mixed well, 1ml per well. Transfection group (lipo2000); in 1.2ml serum-free medium, add 30µl of mRNA at a concentration of 0.1µg/µl and 3µl×3 = 9µl of lipo2000, mix well, let stand for 5-10 min. Cells were washed, transfection reagent was added at 400µl per well, topped up with 600µl DMEM complete medium. Pictures were taken under a fluorescent microscope after 24 hours of treatment.
5. Photo capture: Fluorescence photo: exposure time 600ms, exposure magnification 14, exposure brightness +1; white light photo: exposure time 10ms, exposure magnification 1, exposure brightness +1.

The fluorescence photo results as shown in Figure 3, reveal that cyclic peptide (S5) can carry mRNA into human renal epithelial cells (293T) and express a more pronounced green fluorescent protein compared to the control LNP group, with the fluorescence intensity increasing as the proportion of cyclic peptide increases. At the same time, the cyclic peptide/lipid/mRNA compound formulation (CLS) also showed higher fluorescence expression intensity than the LNP group. Using the Malvern ZetasizerNanoZS, the samples' particle sizes were measured by dynamic light scattering. The particle size distributions of the cyclic peptide/lipid/mRNA compound formulation (CLS) and lipid nanoparticle (LNP) samples are shown in Figure 4. The average particle size of both the CLS and LNP groups were around 100nm with polydispersity indexes (PDI) less than 0.2.

Figure 3 demonstrates that cyclic peptide (S5) can efficiently carry mRNA into 293T cells and express the intended fluorescent protein, with fluorescence intensity increasing along with the increase in cyclic peptide ratio. The cyclic peptide/lipid/mRNA compound formulation (CLS) also exhibits a higher fluorescence expression intensity compared to the control LNP group, indicating efficient nucleic acid delivery into cells.

Figure 4 displays the particle size analysis of the cyclic peptide/lipid/mRNA compound formulation (CLS) and the lipid nanoparticle (LNP) control group samples. Both groups have an average particle size of around 100nm with a polydispersity index (PDI) below 0.2, suggesting that cyclic peptides can combine with nucleic acids to form uniformly sized nanoparticles.

### Embodiment 5: In Vitro Fluorescent Transfection Experiment of Cyclic Peptide/Lipid/mRNA Compound Formulation (CLS).

Optimization of cyclic peptide/lipid/mRNA compound formulation (CLS) for in vitro cell transfection was performed. The preparation and in vitro fluorescence detection were conducted as described in Embodiment 4, with results presented in Figure 5. The cyclic peptide/lipid/mRNA compound formulations were transfected into various cells, including dendritic cells (DC2.4), human non-small cell lung cancer cells (A549), mouse colon cancer cells (CT26), human epidermoid carcinoma cells (A431), human colon cancer cells (RKO), and human renal epithelial cells (293T). The expression intensity of the green fluorescent protein (EGFP) was monitored under a fluorescence microscope 24 hours post-transfection. Remarkable green fluorescence was visible in all cell lines, demonstrating that the cyclic peptide/lipid/mRNA compound formulation (CLS) can effectively carry mRNA into various cells and express the intended fluorescent protein.

Figure 5 shows the efficacy of the cyclic peptide/lipid/mRNA compound formulation (CLS) in carrying mRNA to transfect multiple cell lines and expressing the targeted fluorescent protein. In Figure 5, DC2.4 represents dendritic cells, A549 represents human non-small cell lung cancer cells, CT26 represents mouse colon cancer cells, A431 represents human epidermoid carcinoma cells, RKO represents human colon cancer cells, and 293T represents human renal epithelial cells, with EGFP indicating the green fluorescent protein.

### Embodiment 6: In Vivo Target Gene Knockdown Experiment of Cyclic Peptide/Lipid/siRNA Compound Formulation in Mice.

The experiment utilized 5-week-old female BALB/c mice. The test compounds were administered at a dose of 2 mg/kg through intravenous injection via the tail vein. The liver was collected for target gene expression level analysis 24 hours post-administration.

As shown in Figure 6, lipid nanoparticles (LNP) encapsulating PCSK9-siRNA (MC3/DSPC/Chol/DMG-PEG, molar ratio 50:10:38.5:1.5), cyclic peptide/siRNA particles (CPNP), and lipid/cyclic peptide/siRNA compound particles (LCPNP) were prepared. Upon intravenous injection in mice and liver tissue collection after 24 hours, all three groups exhibited a significant gene knockdown effect for the PCSK9 target gene.

The experiment was repeated with 5-week-old female BALB/c mice, administering the test compounds subcutaneously at a dose of 2 mg/kg. After two administrations, the liver was collected for analysis of the target gene expression level a week later.

The results, as depicted in Figure 7, showed the preparation of LNPs containing PCSK9-siRNA (MC3/DSPC/Chol/DMG-PEG, molar ratio 50:10:38.5:1.5), cyclic peptide/siRNA particles (CPNP), and lipid/cyclic peptide/siRNA compound particles (LCPNP). The mice were given subcutaneous injections for two consecutive days, and liver tissue was obtained on the third day for the determination of PCSK9 target gene expression. Among these, only the LCPNP group showed a marked gene knockdown, whereas the other two groups had some gene silencing effect, but it was not pronounced.

Figures 6 and 7 demonstrate that cyclic peptide/lipid/siRNA compound formulations can effectively deliver siRNA via intravenous and subcutaneous routes in mice, achieving knockdown of the target gene expression.

### Embodiment 7: Transmission Electron Microscopy (TEM) Images of Cyclic Peptide and Lipid/Cyclic Peptide Carrying siRNA.

As illustrated in Figure 8, morphology of the lipid/cyclic peptide/siRNA compound particles under transmission electron microscopy showed an inner layer of smaller nanoparticles encased by a distinct lipid shell.

Figure 8 presents the transmission electron micrograph of the cyclic peptide/lipid/siRNA compound formulation in the form of nanoparticles.

### Embodiment 8: In Vivo Target Fluorescent Protein Expression Experiment of Cyclic Peptide/Lipid/mRNA Compound Formulation (CLS) in Mice.

The experiment employed 6-8 weeks old female C57BL/6 mice. Samples of the cyclic peptide/lipid/mRNA compound formulation (CLS) encapsulating mRNA for expressing luciferase, as well as lipid nanoparticle (LNP) samples, were prepared. Each was administered at a volume of 100µl (0.1µg/µl) via both tail vein and intramuscular injection. At 24 hours post-injection, a luciferin substrate was injected intraperitoneally, followed by in vivo imaging of the animals. Once the substrate had degraded, luciferin was re-injected and mice were dissected. Tissues such as the spleen, liver, and lungs from the intravenously injected group, and the draining lymph nodes from the intramuscularly injected group, were washed with PBS, dried, and subjected to in vivo imaging again.

The fluorescence intensity results within the body at 24 hours post-intravenous injection are shown in Figure 9. Both CLS and LNP groups showed significant fluorescence expression, with the LNP group showing stronger fluorescence. Dissection revealed that fluorescence was primarily concentrated in the spleen and liver tissues.

Results of fluorescence expression within the body at 24 hours post-intramuscular injection are shown in Figure 10. Both CLS and LNP groups exhibited notable fluorescence, with no significant difference in fluorescence intensity at the injection site between the two samples. Post-dissection observation of mouse tissues showed that CLS had stronger fluorescence expression in the lymph nodes at the injection site.

Figures 9 and 10 demonstrate that the cyclic peptide/lipid/mRNA compound formulation (CLS) can effectively deliver mRNA in mice and express the target gene protein.

Table Four provides additional cyclic peptide sequences with amino acid sequences and illustrates these cyclic peptides in the following specific embodiments in conjunction with Table Four.

**Table Four:**

| Serial number | Amino acid sequence | MS | HPLC (%) | Cyclic peptide name |
|---|---|---|---|---|
| SEQ ID No.32 | AAALCKKKKKCLAAA | 1515.90 | 98.41% | CP1-15-A1 |
| SEQ ID No.33 | AGALCKKKKKCLAGA | 1487.55 | 97.88% | CP2-15-AG |
| SEQ ID No.34 | ARLLCKKKKKCLLRA | 1770.23 | 99.04% | CP3-15AR |
| SEQ ID No.35 | ADALCKKKKKCLADA | 1603.45 | 96.80% | CP4-15-D1 |
| SEQ ID No.36 | AAALCKRKRKCLAAA | 1571.55 | 97.01% | CP5-15-R |
| SEQ ID No.37 | AAALCKDKDKCLAAA | 1489.35 | 97.24% | CP6-15-D2 |
| SEQ ID No.38 | AAALCKGKGKCLAAA | 1373.2 | 97.94% | CP7-15-G |
| SEQ ID No.39 | AAALCKAKAKCLAAA | 1401.15 | 95.86% | CP8-15-A2 |
| SEQ ID No.40 | ALLALLCKKKKKKKCLL ALLA | 2308.93 | 97.60% | CP9-21-A |
| SEQ ID No.41 | GSLGSLCKKKKKKCL SGLSG | 2148.53 | 93.38% | CP10-21-GS |
| SEQ ID No.42 | ARLARLCKKKKKKCL RALRA | 2480.83 | 94.34% | CP11-21-R1 |
| SEQ ID No.43 | DLLDLLCKKKKKKKCLL DLLD | 2484.80 | 95.05% | CP12-21-D |
| SEQ ID No.44 | ALLALLCRRKKKRRCLL ALLA | 2420.88 | 96.37% | CP13-21-R2 |
| SEQ ID No.45 | ALLALLCEKEKEKECLL ALLA | 2312.47 | 91.39% | CP14-21-E |
| SEQ ID No.46 | ALLALLCGGKKKGGCL LALLA | 2024.03 | 95.28% | CP15-21-G |
| SEQ ID No.47 | ALLALLCRKRKRKRCLL ALLA | 2420.99 | 92.61% | CP16-21-R3 |
| SEQ ID No.48 | AALLAALLAALLCKKKK KKKKKKKKKKKCLLAA LLAALLAA | 4355.57 | 94.71% | CP17-41-A1 |
| SEQ ID No.49 | GGLLGGLLGGLLCKKK KKKKKKKKKKKCLLG GLLGGLLGG | 4187.2 | 96.87% | CP18-41-G |
| SEQ ID No.50 | DALLDALLDALLCKKKK KKKKKKKKKKCLLAD LLADLLAD | 4619.58 | 90.52% | CP19-41-D1 |
| SEQ ID No.51 | AALLAALLAALLCKDKD KKDKDKKDKDKCLLAA LLAALLAA | 4277.36 | 92.93% | CP20-41-D2 |
| SEQ ID No.52 | AALLAALLAALLCKSKS KKSKSKKSKSKCLLAA LLAALLAA | 4108.77 | 95.22% | CP21-41-S |
| SEQ ID No.53 | AALLAALLAALLCKAKA KKAKAKKAKAKCLLAA LLAALLAA | 4012.72 | 90.86% | CP22-41-A2 |
| SEQ ID No.54 | AALLAALLAALLCRRKR RRRKRRRRKRRCLLAA LLAALLAA | 4691.59 | 94.88% | CP23-41-R1 |
| SEQ ID No.55 | AARLAARLAARLCKKK KKKKKKKKKKKKCLRA ALRAALRAA | 4613.37 | 92.36% | CP24-41-R2 |
| SEQ ID No.56 | AGLLAGLLAGLLAGLLA GLLAGLLCKRKRKKRK RKCLLGALLGALLGALL GALLGALLGA | 5869.06 | 90.53% | CP25-60-GR |
| SEQ ID No.57 | AGLLAGLLAGLLAGLLA GLLAGLLCKGKGKKGK GKCLLGALLGALLGALL GALLGALLGA | 5472.54 | 90.12% | CP26-60-G |
| SEQ ID No.58 | ALGGLALGGLALGGLA LGGLALGGLALGGLCK SKSKSKSKKSKSKSKS KCLGGLALGGLALGGL ALGGLALGGLALGGLA | 7138.18 | 90.30% | CP27-80-GS |
| SEQ ID No.59 | RGDADALCKKKKKCLA | 2260.49 | 99.08% | CP28-RGD |
| | DARGD | | | |
| SEQ ID No.60 | RGDAAALCKDKDKCLA AARGD | 2146.29 | 99.86% | CP29-RGD |
| SEQ ID No.61 | RGDALLALLCEKEKEK ECLLALLARGD | 2969.33 | 97.67% | CP30-RGD |
| SEQ ID No.62 | AGALCKDKRKDCLAGA | 1617.81 | 97.50% | CP31 |
| SEQ ID No.63 | ADALCKRKEKRCLADA | 1789 | 96.00% | CP32 |
| SEQ ID No.64 | AILAILCKKRKKEKKRC LKALKA | 2652.29 | 95.32% | CP33 |
| SEQ ID No.65 | AELVLCKGRDKGKGKG CLVKVA | 2270.68 | 95.60% | CP34 |

### Embodiment 9: Preparation Method of Cyclic Peptides

### Step 1: Preparation of Peptide Resin

1) Select a solid-phase synthesis starting resin according to the target sequence and weigh the respective amount of the starting resin based on substitution level and synthesis scale.
2) Add the weighed starting resin into a 100ml reaction column and swell with 8-10ml of DCM (dichloromethane) or DMF (N,N-dimethylformamide), or a mixture of both per gram of resin. Bubbling with nitrogen gas for approximately 30 minutes, discard the DMF and wash 3 times;
3) Add DBLK (20% piperidine/DMF mixture) solution to the reactor to remove the Fmoc protecting group. To ensure the complete removal of the Fmoc protecting group, deprotection is generally carried out twice.
4) After deprotection of Fmoc, use the ninhydrin test for detection and the result should be positive (a positive result is indicated by a change in color);
5) Following color detection, wash with DMF for 1-2min per wash, with a total of 6 washes;
6) After washing, add the amino acid required for coupling in the target peptide sequence along with solid and liquid condensing reagents to carry out the coupling reaction, for a duration of 1-2 hours;
7) At the end of the reaction, remove the reaction solution and take a small amount of resin for ninhydrin testing which should now give a negative result (a negative result is indicated by no color change). If the result is positive, it shows that there are still exposed amine groups indicating an incomplete reaction. Extra coupling steps should be carried out until the response is complete;
8) Wash 3 times with DMF to remove the washing solution once the step is complete, and repeat steps 3-7 until the last amino acid of the target peptide sequence is coupled;
9) After the completion of the entire peptide sequence coupling and the removal of the Fmoc group from the last amino acid, methanol is used to contract the resin, and drying under vacuum yields the peptide resin.

### Step 2: Preparation of Crude Peptide

1) Weigh the peptide resin from step 1 and cleave it with cleavage reagents to remove the resin carrier and the side-chain protecting groups from the amino acids in the peptide sequence. The proportion and duration of the cleavage reagent depend on the length of the peptide and the complexity of the strategy used to protect the amino acids;
2) After cleavage, filter and retain the filtrate. Precipitate the filtered filtrate by adding it to anhydrous ether at a ratio of 1ml filtrate to 10ml ether for about 20 minutes;
3) After precipitation, centrifuge to remove the supernatant and obtain the filter cake. Wash the filter cake with anhydrous ether, centrifuge, discard the supernatant, and repeat this step 3 times to thoroughly wash the filter cake;
4) Place the washed filter cake in a desiccator and vacuum dry to obtain the crude peptide.

### Step 3: Cyclization of Peptides and Preparation of Final Products

1) Take the crude peptide from step 2, grind it finely and dissolve it in a suitable solvent (water or a mixture of water and acetonitrile) until clear. Cyclization is performed using hydrogen peroxide or iodine.
2) After one or multiple purifications to achieve the target purity, the sample is concentrated by rotary evaporation then freeze-dried under vacuum.
3) The freeze-dried peptide is the final product, which is tested using HPLC (High-Performance Liquid Chromatography) and identified with MS (Mass Spectrometry).

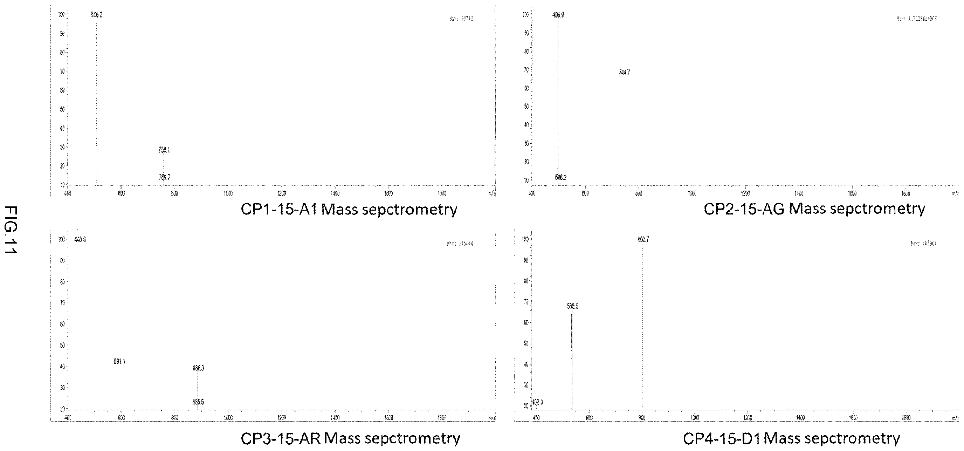

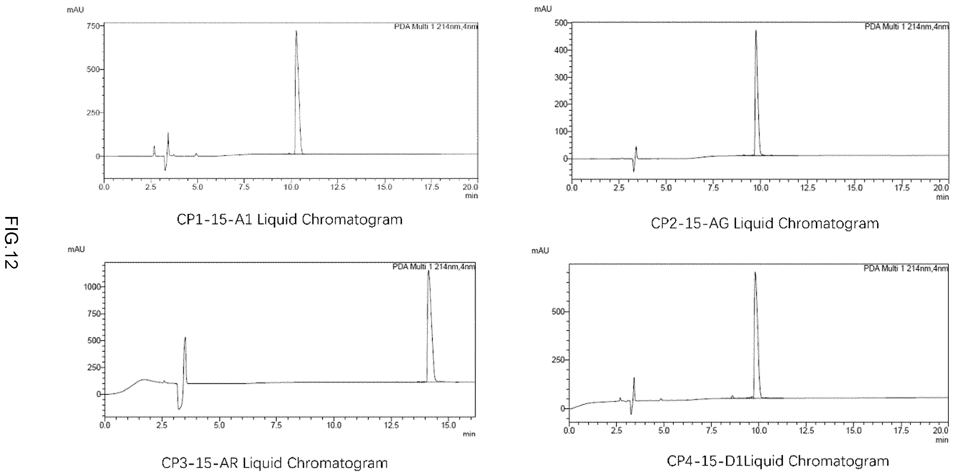

As shown in Figure 11, mass spectrometry displays the relative molecular mass of parts of cyclic peptides, matching the theoretical relative molecular mass. Figure 12 shows that the purity of parts of the cyclic peptides is above 90%, indicating that the synthesized cyclic peptides meet the experimental requirements.

Table Five below provides the names and compositions of CLS formulations prepared and used in Embodiments 10-18. CLS denotes mRNA/cyclic peptide/lipid compound formulation; M denotes mRNA, and L indicates LNP (lipid nanoparticles).

**Table Five: Names and Composition of CLS Formulations**

| CLS Formulation number | Cyclic peptide | Drug capacity |
|---|---|---|
| CLS1 | CP1-15-A1 | 10% |
| CLS2 | CP1-15-A1 | 9% |
| CLS3 | CP1-15-A1 | 7% |
| CLS4 | CP1-15-A1 | 6% |
| CLS5 | CP2-15-AG | 10% |
| CLS6 | CP2-15-AG | 9% |
| CLS7 | CP2-15-AG | 7% |
| CLS8 | CP2-15-AG | 6% |
| CLS9 | CP3-15AR | 10% |
| CLS10 | CP3-15AR | 9% |
| CLS11 | CP3-15AR | 7% |
| CLS12 | CP3-15AR | 6% |
| CLS13 | CP4-15-D1 | 10% |
| CLS14 | CP4-15-D1 | 9% |
| CLS15 | CP4-15-D1 | 7% |
| CLS16 | CP4-15-D1 | 6% |
| CLS17 | CP5-15-R | 10% |
| CLS18 | CP5-15-R | 9% |
| CLS19 | CP5-15-R | 7% |
| CLS20 | CP5-15-R | 6% |
| CLS21 | CP6-15-D2 | 10% |
| CLS22 | CP6-15-D2 | 9% |
| CLS23 | CP6-15-D2 | 7% |
| CLS24 | CP6-15-D2 | 6% |
| CLS25 | CP7-15-G | 10% |
| CLS26 | CP7-15-G | 9% |
| CLS27 | CP7-15-G | 7% |
| CLS28 | CP7-15-G | 6% |
| CLS29 | CP8-15-A2 | 10% |
| CLS30 | CP8-15-A2 | 9% |
| CLS31 | CP8-15-A2 | 7% |
| CLS32 | CP8-15-A2 | 6% |
| CLS33 | CP9-21-A | 10% |
| CLS34 | CP9-21-A | 9% |
| CLS35 | CP9-21-A | 7% |
| CLS36 | CP9-21-A | 6% |
| CLS37 | CP10-21-GS | 10% |
| CLS38 | CP10-21-GS | 9% |
| CLS39 | CP10-21-GS | 7% |
| CLS40 | CP10-21-GS | 6% |
| CLS41 | CP11-21-R1 | 10% |
| CLS42 | CP11-21-R1 | 9% |
| CLS43 | CP11-21-R1 | 7% |
| CLS44 | CP11-21-R1 | 6% |
| CLS45 | CP12-21-D | 10% |
| CLS46 | CP12-21-D | 9% |
| CLS47 | CP12-21-D | 7% |
| CLS48 | CP12-21-D | 6% |
| CLS49 | CP13-21-R2 | 10% |
| CLS50 | CP13-21-R2 | 9% |
| CLS51 | CP13-21-R2 | 7% |
| CLS52 | CP13-21-R2 | 6% |
| CLS53 | CP14-21-E | 10% |
| CLS54 | CP14-21-E | 9% |
| CLS55 | CP14-21-E | 7% |
| CLS56 | CP14-21-E | 6% |
| CLS57 | CP15-21-G | 10% |
| CLS58 | CP15-21-G | 9% |
| CLS59 | CP15-21-G | 7% |
| CLS60 | CP15-21-G | 6% |
| CLS61 | CP16-21-R3 | 10% |
| CLS62 | CP16-21-R3 | 9% |
| CLS63 | CP16-21-R3 | 7% |
| CLS64 | CP16-21-R3 | 6% |
| CLS65 | CP17-41-A1 | 10% |
| CLS66 | CP17-41-A1 | 9% |
| CLS67 | CP17-41-A1 | 7% |
| CLS68 | CP17-41-A1 | 6% |
| CLS69 | CP18-41-G | 10% |
| CLS70 | CP18-41-G | 9% |
| CLS71 | CP18-41-G | 7% |
| CLS72 | CP18-41-G | 6% |
| CLS73 | CP19-41-D1 | 10% |
| CLS74 | CP19-41-D1 | 9% |
| CLS75 | CP19-41-D1 | 7% |
| CLS76 | CP19-41-D1 | 6% |
| CLS77 | CP20-41-D2 | 10% |
| CLS78 | CP20-41-D2 | 9% |
| CLS79 | CP20-41-D2 | 7% |
| CLS80 | CP20-41-D2 | 6% |
| CLS81 | CP21-41-S | 10% |
| CLS82 | CP21-41-S | 9% |
| CLS83 | CP21-41-S | 7% |
| CLS84 | CP21-41-S | 6% |
| CLS85 | CP22-41-A2 | 10% |
| CLS86 | CP22-41-A2 | 9% |
| CLS87 | CP22-41-A2 | 7% |
| CLS88 | CP22-41-A2 | 6% |
| CLS89 | CP23-41-R1 | 10% |
| CLS90 | CP23-41-R1 | 9% |
| CLS91 | CP23-41-R1 | 7% |
| CLS92 | CP23-41-R1 | 6% |
| CLS93 | CP24-41-R2 | 10% |
| CLS94 | CP24-41-R2 | 9% |
| CLS95 | CP24-41-R2 | 7% |
| CLS96 | CP24-41-R2 | 6% |
| CLS97 | CP25-60-GR | 10% |
| CLS98 | CP25-60-GR | 9% |
| CLS99 | CP25-60-GR | 7% |
| CLS100 | CP25-60-GR | 6% |
| CLS101 | CP26-60-G | 10% |
| CLS102 | CP26-60-G | 9% |
| CLS103 | CP26-60-G | 7% |
| CLS104 | CP26-60-G | 6% |
| CLS105 | CP27-80-GS | 10% |
| CLS106 | CP27-80-GS | 9% |
| CLS107 | CP27-80-GS | 7% |
| CLS108 | CP27-80-GS | 6% |
| CLS109 | CP28-RGD | 10% |
| CLS110 | CP28-RGD | 9% |
| CLS111 | CP28-RGD | 7% |
| CLS112 | CP28-RGD | 6% |
| CLS113 | CP29-RGD | 10% |
| CLS114 | CP29-RGD | 9% |
| CLS115 | CP29-RGD | 7% |
| CLS116 | CP29-RGD | 6% |
| CLS117 | CP30-RGD | 10% |
| CLS118 | CP30-RGD | 9% |
| CLS119 | CP30-RGD | 7% |
| CLS120 | CP30-RGD | 6% |
| CLS121 | CP31 | 10% |
| CLS122 | CP31 | 9% |
| CLS123 | CP31 | 7% |
| CLS124 | CP31 | 6% |
| CLS125 | CP32 | 10% |
| CLS126 | CP32 | 9% |
| CLS127 | CP32 | 7% |
| CLS128 | CP32 | 6% |
| CLS129 | CP33 | 10% |
| CLS130 | CP33 | 9% |
| CLS131 | CP33 | 7% |
| CLS132 | CP33 | 6% |
| CLS133 | CP34 | 10% |
| CLS134 | CP34 | 9% |
| CLS135 | CP34 | 7% |
| CLS136 | CP34 | 6% |

### Embodiment 10: Preparation Method of CLS Compound FormulationmRNA Synthesis

mRNA used in the experiments described herein was synthesized in vitro via transcription mediated by T7 polymerase from a linearized DNA template. Utilizing S-Adenosylmethionine (SAM) as the methyl donor, a methyl group was added at the 2'-O position of the first nucleotide adjacent to the 5' cap structure (Cap 0), forming mRNA with a Cap1 structure to increase mRNA translation efficiency. The mRNA was then purified using lithium chloride to remove proteins and most free nucleotides.

LNP Lipid Synthesis: Lipids were dissolved in ethanol at a molar ratio of 50:10:38.5:1.5 (ionizable lipid Dlin-MC3-DMA:DSPC:cholesterol:PEG-lipid).

CLS Formulation: Different dosages of mRNA were uniformly mixed with cyclic peptides and LNP lipid solutions to obtain the CLS compound formulation.

### Embodiment 11: Preliminary In Vitro Cell Transfection Experiment of CLS Compound Formulation

293T cells were seeded at a density of 2×10^4 cells/well in a 96-well cell culture plate and grown for 24 hours until adherence. The CLS formulation was prepared according to the method described in Embodiment 10, and the CLS formulation was added to the cells. After culturing for 24 hours, a color reagent was added and the luminescence of each well was measured using a luminometer. Table six below displays the preliminary screening values of CLS formulations carrying mRNA transfecting 293T cells. The fluorescent expression values show that CLS formulations can effectively carry mRNA into 293T cells and express the target fluorescent protein, indicating that CLS formulations can effectively carry nucleic acids into cells.

**Table Six: Preliminary Cell Screening Transfection Effects of CLS1-CLS108 Formulations**

| CLS Formulation number | RLU |
|---|---|
| CLS1 | 11739254 |
| CLS2 | 10642083 |
| CLS3 | 291063 |
| CLS4 | 93346 |
| CLS5 | 8586880 |
| CLS6 | 7953660 |
| CLS7 | 88219 |
| CLS8 | 59004 |
| CLS9 | 3826154 |
| CLS10 | 4468690 |
| CLS11 | 27798 |
| CLS12 | 40026 |
| CLS13 | 15321015 |
| CLS14 | 18430890 |
| CLS15 | 433018 |
| CLS16 | 178941 |
| CLS17 | 5875816 |
| CLS18 | 7872056 |
| CLS19 | 29501 |
| CLS20 | 31677 |
| CLS21 | 22000288 |
| CLS22 | 20726250 |
| CLS23 | 20327663 |
| CLS24 | 20964532 |
| CLS25 | 6091500 |
| CLS26 | 7439787 |
| CLS27 | 1048634 |
| CLS28 | 7345148 |
| CLS29 | 16877033 |
| CLS30 | 18906669 |
| CLS31 | 16116392 |
| CLS32 | 17175622 |
| CLS33 | 1671523 |
| CLS34 | 1762911 |
| CLS35 | 7843 |
| CLS36 | 13096 |
| CLS37 | 5869635 |
| CLS38 | 5764863 |
| CLS39 | 42730 |
| CLS40 | 81031 |
| CLS41 | 3750315 |
| CLS42 | 3767388 |
| CLS43 | 43905 |
| CLS44 | 20286 |
| CLS45 | 2685298 |
| CLS46 | 3399354 |
| CLS47 | 124234 |
| CLS48 | 161709 |
| CLS49 | 3588266 |
| CLS50 | 3265686 |
| CLS51 | 31784 |
| CLS52 | 26513 |
| CLS53 | 4449239 |
| CLS54 | 5952139 |
| CLS55 | 4861816 |
| CLS56 | 9989323 |
| CLS57 | 2935398 |
| CLS58 | 4920579 |
| CLS59 | 152801 |
| CLS60 | 194901 |
| CLS61 | 4966703 |
| CLS62 | 3750001 |
| CLS63 | 37593 |
| CLS64 | 74918 |
| CLS65 | 1192864 |
| CLS66 | 1316761 |
| CLS67 | 9036 |
| CLS68 | 8839 |
| CLS69 | 12266795 |
| CLS70 | 878702 |
| CLS71 | 18513 |
| CLS72 | 19709 |
| CLS73 | 8597926 |
| CLS74 | 5953613 |
| CLS75 | 160624 |
| CLS76 | 81792 |
| CLS77 | 6912770 |
| CLS78 | 8812530 |
| CLS79 | 3607552 |
| CLS80 | 3525284 |
| CLS81 | 7040797 |
| CLS82 | 3625606 |
| CLS83 | 69751 |
| CLS84 | 28836 |
| CLS85 | 5535954 |
| CLS86 | 5187894 |
| CLS87 | 35261 |
| CLS88 | 49123 |
| CLS89 | 1004974 |
| CLS90 | 932690 |
| CLS91 | 659706 |
| CLS92 | 518153 |
| CLS93 | 4513896 |
| CLS94 | 4507585 |
| CLS95 | 58735 |
| CLS96 | 71034 |
| CLS97 | 804172 |
| CLS98 | 899308 |
| CLS99 | 9648 |
| CLS100 | 32651 |
| CLS101 | 1584795 |
| CLS102 | 1624802 |
| CLS103 | 488402 |
| CLS104 | 653659 |
| CLS105 | 951515 |
| CLS106 | 1143775 |
| CLS107 | 81108 |
| CLS108 | 91776 |
| CLS121 | 279850 |
| CLS122 | 499853 |
| CLS123 | 584290 |
| CLS124 | 594702 |
| CLS125 | 243023 |
| CLS126 | 463763 |
| CLS127 | 541183 |
| CLS128 | 632876 |
| CLS129 | 182774 |
| CLS130 | 347024 |
| CLS131 | 405646 |
| CLS132 | 485661 |
| CLS133 | 160274 |
| CLS134 | 365023 |
| CLS135 | 366455 |
| CLS136 | 448808 |

### Embodiment 12: In Vitro Cell Transfection Experiment of the CLS Compound Formulation

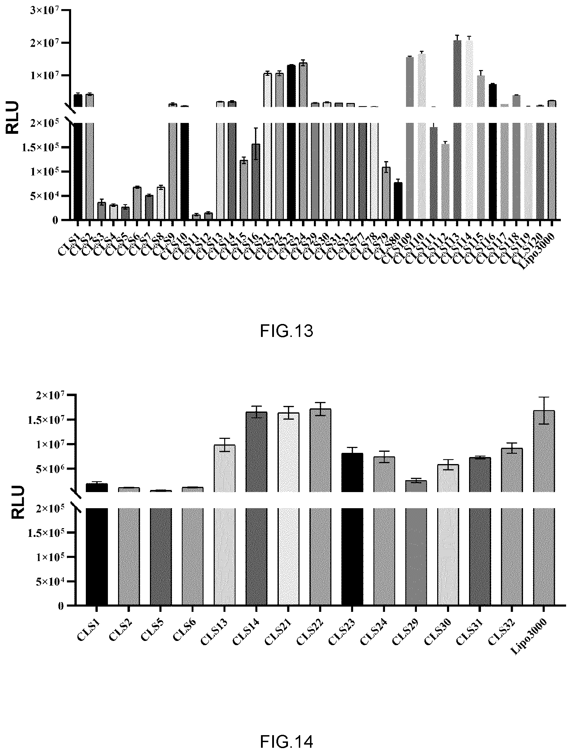

Further selection was made from Embodiment 11 for cyclic peptides that showed good performance-CP1-15-A1, CP2-15-AG, CP3-15-AR, CP4-15-R, CP6-15-D2, CP8-15-A2, CP19-21-A, along with CP28-RGD, CP29-RGD, CP30-RGD - and they were compared with the commercial transfection agent, Lipo3000, for in vitro transfection efficiency. As shown in the results in Figure 13: CLS1, CLS2, CLS9, CLS10, CLS13, CLS14, CLS21, CLS22, CLS23, CLS24, CLS29, CLS30, CLS31, CLS32, CLS33, CLS77, CLS78, CLS117, CLS118, CLS119, CLS120 had transfection effects comparable to Lipo3000, while the formulations of CLS1, CLS2, CLS21, CLS22, CLS23, CLS24, CLS109, CLS110, CLS113, CLS114, CLS115, CLS116 showed better transfection performance than Lipo3000.

### Embodiment 13: Preparation of the CLS Compound Formulation

Different doses of mRNA were uniformly mixed with cyclic peptides and LNP lipid solutions to obtain a compound formulation. Ethanol was then removed by dialysis to give the CLS compound formulation.

### Embodiment 14: Particle Size, Polydispersity Index (PDI), and Zeta Potential of CLS Compound Formulations

The Malvern Zetasizer NanoZS (an instrument for testing nano-particle size) was used to determine the particle size (Size), polydispersity index (PDI), and zeta potential (Zeta P) of particles formed by encapsulating mRNA with CP1-15-A1, CP2-15-AG, CP4-15-R, CP6-15-D2, CP8-15-A2 to form CLS formulations CLS1, CLS2, CLS5, CLS6, CLS13, CLS14, CLS21, CLS22, CLS23, CLS24, CLS29, CLS30, CLS31, CLS32, using dynamic light scattering. Table Seven shows the particle size (Size), polydispersity index (PDI), and zeta potential (Zeta P) values obtained from testing the CLS formulations. As shown in Table Seven: The size of the CLS formulations ranged from 90 to 500nm, with a potential between 10-20mV. CLS1, CLS2, CLS5, CLS6 were larger in size, ranging between 300-500nm, whereas the CLS13, CLS14, CLS21, CLS22, CLS23, CLS24, CLS29, CLS30, CLS31, CLS32 formulations had particle sizes around 100nm. The polydispersity indexes varied around 0.1-0.3.

**Table Seven: Particle Size (Size), Polydispersity Index (PDI), and Zeta Potential (Zeta P) of CLS Formulations**

| CLS Formulation number | Size | PDI | Zeta P |
|---|---|---|---|
| CLS1 | 474.17 | 0.132 | 14.133 |
| CLS2 | 381.7 | 0.111 | 17.733 |
| CLS5 | 561.53 | 0.149 | 15 |
| CLS6 | 400.73 | 0.096 | 11.933 |
| CLS13 | 243.5 | 0.322 | 16.3667 |
| CLS14 | 149.8 | 0.174 | 11.2 |
| CLS21 | 103.53 | 0.219 | 16.4 |
| CLS22 | 125.27 | 0.393 | 18.067 |
| CLS23 | 174.53 | 0.226 | 17.967 |
| CLS24 | 122.4 | 0.154 | 21.6 |
| CLS29 | 130.07 | 0.24 | 16.333 |
| CLS30 | 98.52 | 0.1696 | 16.567 |
| CLS31 | 126.8 | 0.217 | 14.933 |
| CLS32 | 114.1 | 0.151 | 12.3 |

### Embodiment 15

According to the preparation method of CLS compound formulation described in Embodiment 13, CLS1, CLS2, CLS5, CLS6, CLS13, CLS14, CLS21, CLS22, CLS23, CLS24, CLS29, CLS30, CLS31, CLS32 nanoparticles were prepared and used for cell transfection experiments. The results are shown in Figure 14, indicating that CLS13, CLS14, CLS21, CLS22, CLS30, CLS31, CLS32 had superior transfection effects compared to other formulations.

### Embodiment 16: Gel Electrophoresis Encapsulation Experiment

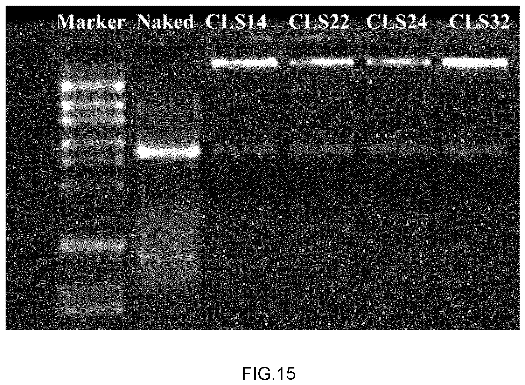

First, a 1.5% gel was prepared by heating the agarose solution in a microwave until fully dissolved, then adding SYBR green dye before pouring it into a gel casting tray with the comb inserted. CLS formulations and bare mRNA were thoroughly mixed with the loading buffer at a 1:1 ratio. The Marker, bare mRNA, and CLS formulations were added to the gel wells. An electric voltage of 190 volts was applied for about 20 minutes, and images were captured with a gel imaging system. As shown in Figure 15, the results showed that all CLS carriers encapsulated mRNA effectively.

### Embodiment 17: In Vivo Expression Experiment of Target Fluorescent Protein after Intravenous Injection of CLS Compound Formulation in Mice

The experiment used 6-8-week-old female SPF-grade BALB/c mice (n=3), housed in an SPF-grade facility with 12/12 hours light/dark cycle, temperature 20-26°C, humidity 40-70%, with ample feed and water. A dose of 100µL (0.05µg/µL) was administered intravenously using CLS14, CLS22, CLS24, and CLS32 formulations prepared according to the method in Embodiment 13. Lipid MC3 served as the standard control, and MC3-LNP was used for in vivo efficacy comparison in the control group. A negative control was treated with PBS. Six hours post-administration, the mice received an intraperitoneal injection of 200µL of 15mg/mL D-luciferin potassium salt, placed on an imaging table, and live imaging was conducted 5 minutes after the D-luciferin injection. After completing live imaging for all groups, a second injection of D-luciferin was administered, and 5 minutes later, the mice were dissected, and their organs (heart, liver, spleen, lung, kidney) were imaged.

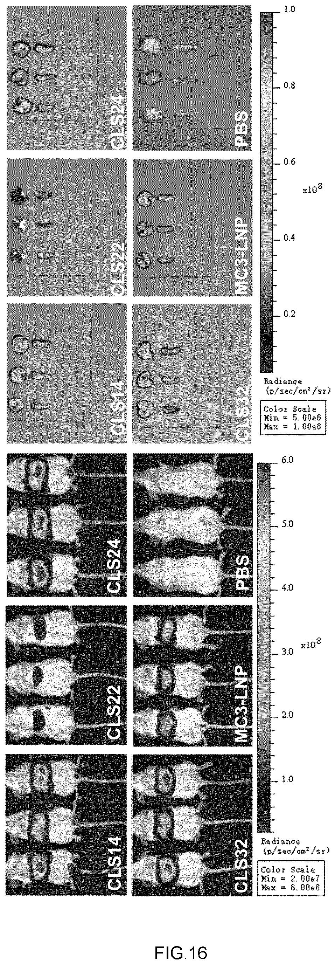

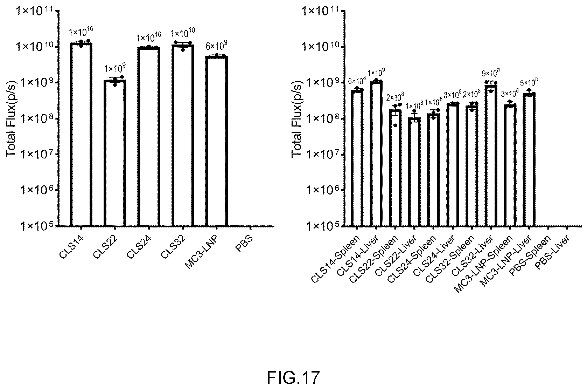

As shown in Figures 16 and 17: Both the CLS groups and MC3-LNP group expressed significant fluorescence in mice. Notably, the fluorescence expression in CLS14, CLS24, and CLS32 groups was stronger, substantially superior to the MC3-LNP control group. After dissection, it was observed that fluorescence was mainly concentrated in the spleen and liver tissues. This demonstrates that the CLS formulations can effectively deliver mRNA and efficiently express the target gene protein in mice.

### Embodiment 18: In Vivo Expression of the Target Fluorescent Protein after Intramuscular Injection of the CLS Compound Formulation in Mice

The experiment was carried out on 6-8-week-old female SPF-grade BALB/c mice (n=3), housed in an SPF-grade facility with a 12/12-hour light/dark cycle, temperature 20-26°C, and humidity 40-70%, with ample feed and water. A 100µL (0.05µg/µL) dose was administered intramuscularly using CLS formulations CLS14, CLS24, and CLS32 prepared according to the method described in Embodiment 13. Lipid MC3, the current standard in the field, was used as the control, and MC3-LNP was utilized for in vivo efficacy comparison. The negative control used PBS. Six hours after administration, the mice were injected intraperitoneally with 200µL of 15mg/mL D-luciferin potassium salt. The mice were then placed on an imaging table for in vivo imaging, 5 minutes after the D-luciferin injection. After completing live imaging for all groups, D-luciferin was injected again, and 5 minutes later, the mice were dissected, and their organs (heart, liver, spleen, lungs, kidneys) were imaged.

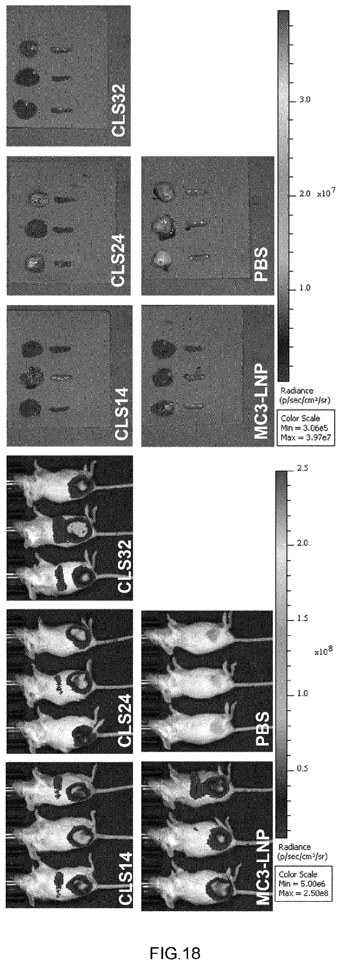

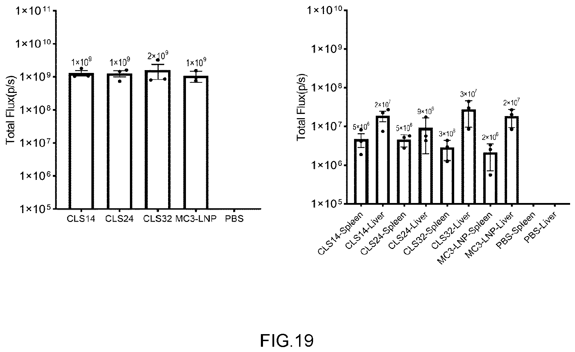

As shown in Figures 18 and 19: Both the CLS groups and the MC3-LNP group showed significant expression of fluorescence in mice, with the CLS32 group exhibiting stronger fluorescence, significantly surpassing the MC3 control group. Upon observing the mouse organ tissues, the fluorescence intensity of the CLS group was found to be comparable to the MC3 control group. This indicates that the CLS formulation can effectively deliver mRNA and efficiently express the target gene protein within mice.

In summary: The preparation process of the cyclic peptide or cyclic peptide composition described in this invention is controllable and effectively delivers nucleic acids to cells, tissues, or organisms. It can effectively carry nucleic acid drugs into the cells within tissues and organs of the body. The in vitro experiments have comparable transfection effects to commercial transfection agents, and in vivo, it is comparable to MC3-LNP. Therefore, the cyclic peptide has great potential for nucleic acid delivery. In the descriptions of this specification, referential terms such as "an embodiment," "some embodiments," "an example," "a specific example," or "some examples" indicate that the particular features, structures, materials, or characteristics described in combination with the embodiment or example are included in at least one embodiment or example of the invention. In this document, the schematic representation of such terms does not necessarily refer to the same embodiment or example. Also, the described specific features, structures, materials, or characteristics can be combined in any one or more embodiments or examples in an appropriate manner. Furthermore, those skilled in the art can combine and combine different disclosed embodiments or examples according to the descriptions in this document.

Although the embodiments of the invention have been shown and described, it will be understood that the above embodiments are exemplary and should not be construed as limiting the invention. Persons skilled in the art may make variations, modifications, and variations to the above-described embodiments within the scope of the invention.

## Claims

1. A cyclic peptide with a following sequence general formula:
P-(X)m-Cys-(Y)n-Cys-(Z)o-P;
wherein,
X=Xaa₁-Xaa₂-Leu, or Xaa₁-Xaa₂-Xaa₃-Leu, or Xaa₁-Xaa₂-Xaa₃-Xaa₄-Leu,
Y=Xaa₅-Xaa₆-Lys-Xaa₆-Xaa₅, or Xaa₅-Xaa₆-Xaa₇-Lys-Xaa₇-Xaa₆-Xaa₅, or Xaa₅-Xaa₆-Xaa₇-Xaa₈-Lys-Xaa₈-Xaa₇-Xaa₆-Xaa₅,
Z=Leu-Xaa₂-Xaa₁, or Leu-Xaa₃-Xaa₂-Xaa₁, or Leu-Xaa₄-Xaas-Xaa₂-Xaa₁,
Xaa₁, Xaa₂, Xaa₃, Xaa₄, Xaa₅, Xaa₆, Xaa₇, and Xaa₈ are any one of alanine (Ala), valine (Val), leucine (Leu), isoleucine (Ile), proline (Pro), phenylalanine (Phe), tryptophan (Trp), methionine (Met), glycine (Gly), serine (Ser), tyrosine (Tyr), threonine (Thr), asparagine (Asn), glutamine (Gln), aspartic acid (Asp), glutamic acid (Glu), lysine (Lys), or arginine (Arg);
P is an optional ligand that may or may not be present and can be independently chosen from agents such as spermine or spermidine, polylysine, polyarginine, MPG peptide, HIV-binding peptide Tat, SAP peptide, MAP peptide, KALA peptide, FGF peptide, HSV peptide, RGD peptide, GLP-1 peptide, Pep-1 peptide, signal peptides or signal sequences, cytokines, growth factors, hormones, small molecule drugs, carbohydrates, therapeutic active proteins or peptides, antigens or epitopes, antibodies, ligands for antibody receptors, synthetic ligands, inhibitors or antagonists of receptors, immunostimulatory proteins or peptides;
a disulfide bond is formed between two cysteines (Cys);
m, n, or o can independently be any number selected from 0, 1, 2, 3, 4, 5, 6, 7, 8, 9, or 10;
the cyclic peptide comprises amino acid residues having a length from 15-150.

2. A cyclic peptide complex, **characterized in that**, comprising one or more cyclic peptides as claimed in claim 1, wherein the general formula of the cyclic peptide is: P-(X)m-Cys-(Y)n-Cys-(Z)o-P, and a disulfide bond is formed between the two cysteines (Cys).

3. A cyclic peptide and drug composition, **characterized in that**, the cyclic peptide has a peptide structure claimed in claims 1 or 2, the cyclic peptide structure is capable of carrying nucleic acid molecules, therapeutic drugs, or agents to form a composition, the drugs or agents selected from one or more among peptides, antibodies, enzymes, hormones, antitumor drugs, antilipidemic drugs, antibiotics, anti-inflammatory agents, cytotoxins, cell growth inhibitors, immunoregulators or neuroactive agents.

4. The cyclic peptide and drug composition according to claim 3, **characterized in that** the carried nucleic acid comprises one or more selected from DNA or RNA, encoding DNA, encoding RNA, encoding mRNA, small interfering nucleic acids siRNA, small activating nucleic acids saRNA, micro nucleic acids microRNA, messenger nucleic acids mRNA, antisense nucleic acids, or immunostimulatory nucleic acids.

5. The cyclic peptide and drug composition according to claim 3, **characterized in that** the composition is used in combination with one or more of surfactants, liposomes, liposome-like bodies, alcosomes, transporters, phospholipid mixtures.

6. The cyclic peptide and drug composition according to claim 5, **characterized in that** the liposomes comprise one or more commercial liposomes such as N-(2,3-dioleyloxy)propyl)-N,N,N-trimethylammonium chloride (DOTAP), N-(2,3-diacyloxy)propyl)-N,N,N-trimethylammonium chloride (DOTMA), distearoylphosphatidylcholine (DSPC), dioleoylphosphatidylcholine (DOPC), dipalmitoylphosphatidylcholine (DPPC), dioleoylphosphatidylglycerol (DOPG), dioleoyl-phosphatidylethanolamine (DOPE), and others.

7. The cyclic peptide and drug composition according to claim 6, **characterized in that** the cyclic peptide or the cyclic peptide and drug composition can form complex particles with one or more of the liposomes.

8. The cyclic peptide and drug composition according to claim 3, **characterized in that** the cyclic peptide or the cyclic peptide and drug composition can be used together with one or more pharmaceutically acceptable carriers, buffers, diluents, or excipients.

9. Use of a cyclic peptide structure or a cyclic peptide and drug composition defined according to any one of claims 1 to 3 in a preparation for prevention, treatment, and/or improvement of selected diseases from following list: cancer or tumor diseases, viral or bacterial infectious diseases, genetic diseases, respiratory system diseases, cardiovascular diseases, neurological diseases, digestive system diseases, skeletal diseases, connective tissue diseases, immune deficiency diseases, endocrine diseases, eye and ear diseases.

10. Use of a cyclic peptide structure or a cyclic peptide and drug composition defined according to any one of claims 1 to 3 in a preparation for prevention, treatment, and/or improvement of following animal diseases, for veterinary medicinal compositions, or for animal vaccines.

11. A cyclic peptide with a following sequence general formula:
P-(X)m-Cys-(Y)n-Cys-(Z)o-P;
wherein,
X = Xaa₁-Xaa₂-Leu, or Xaa₁-Xaa₂-Xaa₃-Leu, or Xaa₁-Xaa₂-Xaa₃-Xaa₄-Leu;
Y = a peptide fragment composed of various amino acids, with the number of Lys in Y being 1-20;
Z = Leu-Xaa₅-Xaa₆, or Leu-Xaa₅-Xaa₆-Xaa₇, or Leu-Xaa₅-Xaa₆-Xaa₇-Xaa₈;
Xaa₁, Xaa₂, Xaa₃, Xaa₄, Xaa₅, Xaa₆, Xaa₇, and Xaa₈ are any one of alanine (Ala), valine (Val), leucine (Leu), isoleucine (Ile), proline (Pro), phenylalanine (Phe), tryptophan (Trp), methionine (Met), glycine (Gly), serine (Ser), tyrosine (Tyr), threonine (Thr), asparagine (Asn), glutamine (Gln), aspartic acid (Asp), glutamic acid (Glu), lysine (Lys), or arginine (Arg);
Xaax is a peptide fragment composed of one or more amino acids selected from alanine (Ala), valine (Val), leucine (Leu), isoleucine (Ile), proline (Pro), phenylalanine (Phe), tryptophan (Trp), methionine (Met), glycine (Gly), serine (Ser), tyrosine (Tyr), threonine (Thr), asparagine (Asn), glutamine (Gln), aspartic acid (Asp), glutamic acid (Glu), lysine (Lys), or arginine (Arg);
P is an optional ligand that may or may not be present and can be independently selected from agents such as spermine or spermidine, polylysine, polyarginine, MPG peptide, HIV-binding peptide Tat, SAP peptide, MAP peptide, KALA peptide, FGF peptide, HSV peptide, RGD peptide, GLP-1 peptide, Pep-1 peptide, signal peptides or signal sequences, cytokines, growth factors, hormones, small molecule drugs, carbohydrates, therapeutic active proteins or peptides, antigens or epitopes, antibodies, ligands for antibody receptors, synthetic ligands, inhibitors or antagonists of receptors, immunostimulatory proteins or peptides;
a disulfide bond is formed between two cysteines (Cys);
m, n, or o can each be independently selected from any one number from 0, 1, 2, 3, 4, 5, 6, 7, 8, 9, or 10;
the cyclic peptide comprises amino acid residues having a length from 15-150.

12. A cyclic peptide complex **characterized by** comprising one or more cyclic peptides as claimed in claim 11, wherein the general formula of the cyclic peptide is as follows:
P-(X)m-Cys-(Y)n-Cys-(Z)o-P, and a disulfide bond is formed between two cysteines (Cys).

13. A cyclic peptide and drug composition, **characterized in that**, the cyclic peptide has a peptide structure claimed in claims 11 or 12; the cyclic peptide structure is capable of carrying nucleic acid molecules, therapeutic drugs, or agents to form a composition, the drugs or agents selected from one or more among peptides, antibodies, enzymes, hormones, antitumor drugs, antilipidemic drugs, antibiotics, anti-inflammatory agents, cytotoxins, cell growth inhibitors, immunoregulators or neuroactive agents.

14. The cyclic peptide and drug composition according to claim 13, **characterized in that** the carried nucleic acid comprises one or more selected from DNA or RNA, encoding DNA, encoding RNA, encoding mRNA, small interfering nucleic acids siRNA, small activating nucleic acids saRNA, micro nucleic acids microRNA, messenger nucleic acids mRNA, antisense nucleic acids, or immunostimulatory nucleic acids.

15. The cyclic peptide and drug composition according to claim 13, **characterized in that** the composition is used in combination with one or more of following surfactants, liposomes, liposome-like bodies, alcosomes, transporters, phospholipid mixtures.

16. The cyclic peptide and drug composition according to claim 15, **characterized in that** the liposomes comprise one or more commercial liposomes such as N-(2,3-dioleyloxy)propyl)-N,N,N-trimethylammonium chloride (DOTAP), N-(2,3-dioleoyloxy)propyl)-N,N,N-trimethylammonium chloride (DOTMA), distearoylphosphatidylcholine (DSPC), dioleoylphosphatidylcholine (DOPC), dipalmitoylphosphatidylcholine (DPPC), dioleoylphosphatidylglycerol (DOPG), dioleoyl-phosphatidylethanolamine (DOPE), among others.

17. The cyclic peptide and drug composition according to claim 16, **characterized in that** the cyclic peptide or the cyclic peptide and drug composition are capable of forming complex particles with one or more of the liposomes.

18. The cyclic peptide and drug composition according to claim 13, **characterized in that** the cyclic peptide or the cyclic peptide and drug composition can be used together with one or more pharmaceutically acceptable carriers, buffers, diluents, or excipients.

19. Use of a cyclic peptide structure or a cyclic peptide and drug composition defined according to any one of claims 11 to 13 in a preparation for prevention, treatment, and/or improvement of selected diseases from following list: cancer or tumor diseases, viral or bacterial infectious diseases, genetic diseases, respiratory system diseases, cardiovascular diseases, neurological diseases, digestive system diseases, skeletal diseases, connective tissue diseases, immune deficiency diseases, endocrine diseases, eye and ear diseases.

20. Use of a cyclic peptide structure or a cyclic peptide and drug composition defined according to any one of claims 11 to 13 in a preparation for prevention, treatment, and/or improvement of following animal diseases, for veterinary medicinal compositions, or for animal vaccines.
